# EUROPEAN PATENT SPECIFICATION

(11) **EP 3 558 268 B1**
(45) Date of publication and mention of the grant of the patent: **03.09.2025**
(21) Application number: 17884892.5
(22) Date of filing: 21.12.2017
(51) Int. Cl.: A61K 9/10, A61K 9/52, A61K 31/198, A61K 9/00, A61K 9/16, A61K 9/22, A61K 9/50, A61P 3/00, A61P 9/00, A61P 25/04, A61P 25/16, A61P 25/28, A61P 13/00, A61K 47/36

(54) **DROXIDOPA COMPOSITIONS AND METHODS**
DROXIDOPAZUSAMMENSETZUNGEN UND VERFAHREN
COMPOSITIONS DE DROXIDOPA ET MÉTHODES

(30) Priority: 22.12.2016 US 201662438056 P
(43) Date of publication of application: 30.10.2019
(73) Proprietor: Xenamed Corp., St. Paul, Minnesota 55114 (US)
(72) Inventor: DANDIKER, Yogesh, Edina Minnesota 55436 (US); PANCHAL, Maulik Kiritkumar, Maple Grove Minnesota 55311 (US); YU, Xiao, Maple Grove Minnesota 55311 (US); NELSON, Patrick, Bloomington Minnesota 55431 (US)
(74) Representative: Nederlandsch Octrooibureau
(86) International application number: PCT/US2017/068038
(87) International publication number: WO 2018/119323

(56) References cited:
- US-A1- 2012 029 085
- US-A1- 2013 243 875
- US-A1- 2013 243 875
- US-B2- 8 865 692
- THANGARATHINAM KUMAR ET AL: "Stability-Indicating Related Substances HPLC Method for Droxidopa and Characterization of Related Substances Using LC-MS and NMR", JOURNAL OF CHROMATOGRAPHIC SCIENCE, vol. 54, no. 10, 6 September 2016 (2016-09-06), Cary, NC, USA, pages 1761 - 1770, XP055711037, ISSN: 0021-9665, DOI: 10.1093/chromsci/bmw136

## Description

### CROSS-REFERENCE TO RELATED APPLICATIONS

This application claims priority to U.S. Provisional Patent Application No. 62/438,056, filed on December 22, 2016.

### BACKGROUND

Droxidopa is a synthetic amino acid precursor which acts as a prodrug to the neurotransmitters norepinephrine (noradrenaline) and epinephrine (adrenaline) and which is used to increase the concentrations of these neurotransmitters in the body and brain. Chemically, droxidopa is (-)-threo-3-(3,4-Dihydroxyphenyl)-L-serine, and has the following structural formula:

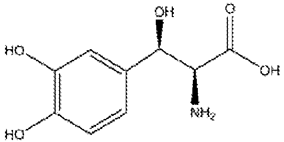

Unlike norepinephrine and epinephrine themselves, droxidopa is capable of crossing the protective blood-brain barrier (BBB). It is metabolized by aromatic L-amino acid decarboxylase (AAAD), also known as DOPA decarboxylase (DDC).

Droxidopa is used to treat neurogenic orthostatic hypotension. Neurogenic orthostatic hypotension has a variety of causes and is also a common symptom of Parkinson's disease. Droxidopa is thought to work by increasing the levels of norepinephrine and epinephrine in the peripheral nervous system (PNS), which induces tachycardia or increased heart rate and hypertension or increased blood pressure, thus enabling the body to maintain blood flow upon and while standing.

The droxidopa dosage form currently approved for use in the United States is immediate release oral capsules comprising 100, 200, or 300 mg droxidopa, with a maximum daily dosage limited to 1800 mg. Current dosing regimens are administration of one or two capsules three times daily.

### SUMMARY

Disclosed, in various embodiments, are oral dosage forms comprising droxidopa, or a pharmaceutically acceptable salt thereof, as defined below.

In some aspects, the disclosure is directed to a pharmaceutical composition comprising droxidopa, or a pharmaceutically acceptable salt thereof, for oral administration, wherein the pharmaceutical composition is an extended release liquid dosage form or solid dosage form as further defined in claims 1 or 7. In some embodiments, the liquid dosage form is a suspension. In some embodiments, the solid dosage form comprises a solid composition packaged in a bottle, a sachet, or a packet for suspension in a liquid vehicle.

In a first aspect, the present invention is directed to an extended-release liquid composition for oral administration comprising:
(a)
   (i) a first multi-particulate comprising droxidopa, or a pharmaceutically acceptable salt thereof, and optionally a first release controlling agent; and
   (ii) a second multi-particulate comprising droxidopa, or a pharmaceutically acceptable salt thereof, a second release controlling agent, and an enteric layer; and
(b) a liquid vehicle,
wherein the first multi-particulate is capable of releasing droxidopa, or a pharmaceutically acceptable salt thereof, at a pH of less than 1.5, and the second multi-particulate is capable of releasing droxidopa, or a pharmaceutically acceptable salt thereof, at a pH of 5.5.

In some embodiment, the release controlling agent is a non-polymeric material. In some embodiments, the multi-particulate is in the form of pellets, granulates, or a mini-tablets. The multi-particulate comprises two or more types of pellets, e.g., one type can be immediate release pellets whereas another type can release the drug over an extended period. The multi-particulate comprises two types of extended release pellets with each type having a different release profile. At least one type of the pellets is coated. In some embodiments, all types of of the pellets are coated.

In some embodiments, the liquid vehicle has a pH of less than 7.0. In some embodiments, the liquid vehicle includes a buffering agent.

In an embodiment, an extended-release suspension or suspension for oral administration includes an extended-release multi-particulate including an effective amount of droxidopa, or a pharmaceutically acceptable salt thereof, and a release-controlling agent; and a suspending vehicle, wherein the suspension has a pH < 7.0.

In a second aspect, the present invention is directed to a solid pharmaceutical composition for oral administration comprising
(i) a first multi-particulate comprising droxidopa, or a pharmaceutically acceptable salt thereof, and optionally a first release controlling agent; and
(ii) a second multi-particulate comprising droxidopa, or a pharmaceutically acceptable salt thereof, a second release controlling agent, and an enteric layer,
wherein the first multi-particulate is capable of releasing droxidopa, or a pharmaceutically acceptable salt thereof, at a pH of less than 1.5, and the second multi-particulate is capable of releasing droxidopa, or a pharmaceutically acceptable salt thereof, at a pH of 5.5. composition for oral administration includes an extended-release multi-particulate including an effective amount of droxidopa, or a pharmaceutically acceptable salt thereof, and a non-polymeric release-controlling agent. In some embodiments, the solid pharmaceutical composition is suitable for suspension in a liquid vehicle.

In an embodiment, a pharmaceutical kit includes a solid pharmaceutical composition disclosed herein and a liquid vehicle. In some embodiments, the pharmaceutical kit comprises a solid pharmaceutical composition disclosed herein in a container such as a sachet or a bottle. In some embodiments, the disclosure is directed to a pharmaceutical kit comprising: (a) a solid composition comprising: (i) a first multi-particulate comprising droxidopa, or a pharmaceutically acceptable salt thereof, and optionally, a first release controlling agent and (ii) a second multiparticulate comprising droxidopa, or a pharmaceutically acceptable salt thereof, and a second release controlling agent; and (b) a liquid vehicle. In some embodiments, the pharmaceutical kit comprises (a) an extended-release multi-particulate comprising an effective amount of droxidopa, or a pharmaceutically acceptable salt thereof, and a release-controlling agent; and (b) an immediate-release composition (e.g., a capsule) comprising an effective amount of droxidopa.

Methods of making the dosage form are disclosed but not claimed. Any method of treatment mentioned herein is to be construed as compositions for use in a method of treatment.

In a further aspect, the composition or dosage form of the claimed invention is for use in treating a subject in need of treatment of hypotension, neurogenic orthostatic hypotension (nOH), intradialytic hypotension, a symptom of Parkinson's disease, orthostatic hypotension associated with Parkinson's disease, postural instability associated with Parkinson's disease, postural orthostatic tachycardia syndrome (POTS), Down's syndrome, a demyelinating disease, Alzheimer's disease, an attention deficit disorder, hypersomnia, pain associated with fibromyalgia, motor paralysis, motor aphasia, urinary incontinence, dementia, antidiuresis, postural tachycardia syndrome, tauopathy, fatigue, headaches, neurological deficits or neuronal death induced by brain ischemia, intracranial hypertension or cerebral edema, cancer, a bacterial infection, to induce or facilitate micturition, nasal congestion, acute pain, chronic pain, or any combination thereof.

These and other features and characteristics are more particularly described below.

### BRIEF DESCRIPTION OF THE DRAWINGS

The following is a brief description of the drawings which are presented for the purposes of illustrating the exemplary embodiments disclosed herein and not for the purposes of limiting the same.
FIG. 1 provides flow-charts for six different processes to manufacture extended release multi-particulates: hot-melt extrusion, melt-granulation, extrusion-spheronization, direct pelletization, and spray drying/spray congealing.

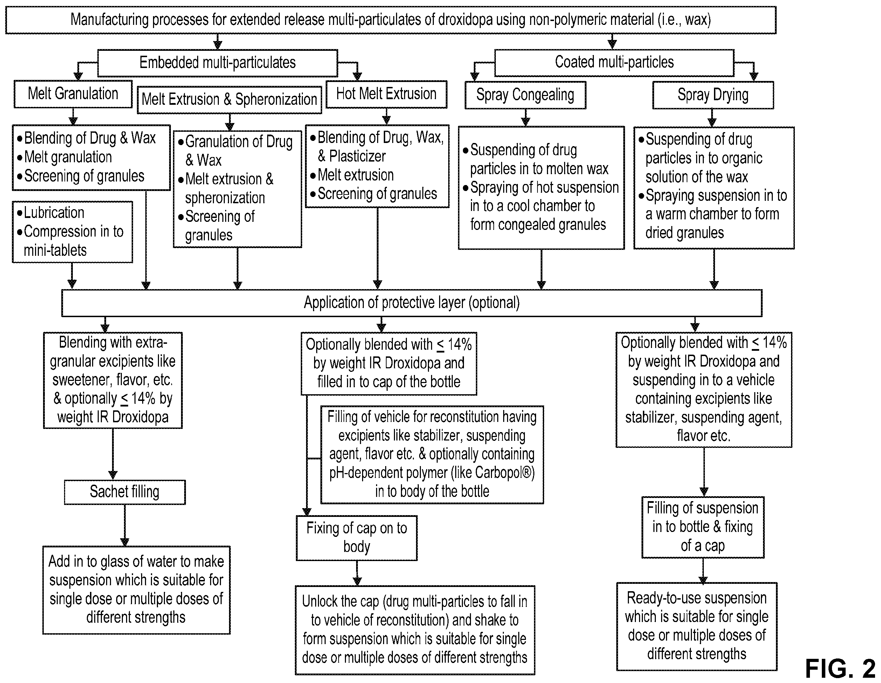

FIG. 2 is a flow chart for manufacture of three different embodiments of the oral dosage forms: a sachet comprising extended release multi-particulates for addition to a liquid vehicle to form a suspension, a kit for making a suspension in the form of a bottle with the extended release multi-particulates within a sealed cap and the liquid vehicle in the bottle, and a single or multiple dose ready-to-use suspension in a bottle or vial.
FIG. 3 shows an *in vitro* dissolution profile (% release over time) for a droxidopa extended-release multi-particulate suspension where the dissolution was performed in 0.1N HCl for the two hour and in pH 6.8 phosphate buffer for 2 to 8 hours.
FIG. 4 shows an *in vitro* dissolution profile (% release over time) for droxidopa extended-release granules prepared by melt granulation process where the dissolution was performed in 0.1N HCl for 0 to 2 hours and in pH 6.8 phosphate buffer for 2 to 14 hours.
FIG. 5 shows an *in vitro* dissolution profile (% release over time) for a 900 mg extended-release mini-tablets where the dissolution was performed in 0.1N HCl for 0 to 2 hours and in pH 6.8 phosphate buffer for 2 to 8 hours.
FIG. 6 shows an *in vitro* dissolution profile (% release over time) for pulsatile release formulation I where the dissolution was performed in 0.1N HCl for 0 to 2 hours and in pH 6.8 phosphate buffer for 2 to 12 hours.
FIG. 7 shows an *in vitro* dissolution profile (% release over time) for pulsatile release formulation II where the dissolution was performed in 0.1N HCl for 0 to 2 hours and in pH 6.8 phosphate buffer for 2 to 12 hours.
FIG. 8 shows one example of the pharmaceutical kit disclosed herein, in the form of a capped bottle comprising a solid dosage form and a liquid vehicle for reconstituting the solid dosage form.

### DETAILED DESCRIPTION

Currently approved dosing regimens for droxidopa are administration of one or two capsules three times daily. Patients typically treated with droxidopa, for example Parkinson's patients, have difficulty with vertical stability and/or difficulty swallowing large capsules. Patients administered capsules can also feel uncomfortable because capsules can stick to their throat. Three times daily dosing is inconvenient and can decrease patient compliance. To address these problems, the inventors have developed alternative oral dosage forms that are easier and more comfortable to swallow, and/or whose release properties permit dosing fewer than three times daily. This applicaton discloses extended release droxidopa dosage forms, e.g., a solid dosage form that can be reconstituted to a liquid dosage form or a ready-to-use liquid dosage form. In some embodiments, the pharmaceutical compositions disclosed herein allow for oral administration only once or twice daily.

Disclosed herein are improved oral dosage forms comprising droxidopa such as, for example, extended-release formulations, which are more easily administered than currently marketed dosage and require fewer dosage forms to be administered daily.

In one aspect, the extended release dosage form is a solid pharmaceutical composition. The composition comprises an extended-release multi-particulate comprising an effective amount of droxidopa, or a pharmaceutically acceptable salt thereof, and optionally a non-polymeric release-controlling agent.

In another aspect, the extended release dosage form is a suspension for oral administration. The suspension can comprise an extended-release multi-particulate comprising an effective amount of droxidopa, or a pharmaceutically acceptable salt thereof, and a release-controlling agent; and a suspending vehicle, wherein the suspension has a pH < 7.0.

In some embodiments, the suspension has a pH less than 5.0. In some embodiments, the suspension has a pH of from about 2.0 to about 5.0, or from about 3.0 to about 5.0. In some embodiments, the suspension has a pH of about 4.0.

### Multi-particulates

The compositions or oral dosage forms disclosed herein comprise a first and a second multi-particulate (used interchangeably herein with "multi-particulates"), e.g., an extended release multi-particulate. Multi-particulates are discrete, small drug units, exhibiting a desired characteristic, that make up a multiple unit drug delivery system. The multi-particulates can be in the form of, for example, a drug particle, a granule, a pellet, a bead, a sphere, or a mini-tablet. Any of these multi-particulate forms can be coated or uncoated. In some embodiments, a desired characteristic of the multi-particulates is particle size, e.g., less than about 400 µm. In another embodiment, a desired characteristic of the multi-particulates is controlled release (e.g., extended release) of the drug (e.g., droxidopa or a pharmaceutically acceptable salt thereof) over a period of time.

The multi-particulate comprises droxidopa or a pharmaceutically acceptable salt thereof. At least the second multi-particulate comprises a release controlling agent. In some embodiments, the multi-particulate comprises a wax/lipid matrix (e.g., the multi-particulate are wax/lipid embedded matrix pellets). In some embodiments, the droxidopa is about 10% to about 70% (e.g. about 15% to about 35%) of the total weight of the multi-particulate. In some embodiments, the droxidopa is about 20% of the total weight of the multi-particulate.

The extended release multi-particulate can further comprise an excipient, e.g., a lubricant, a binder, a filler, a glidant, a plasticizer, or a combination thereof. Examples of suitable lubricants include sodium stearyl fumarate, stearic acid, magnesium stearate, glyceryl behenate, talc, and combinations comprising one or more of the foregoing lubricants. Examples of suitable binders include water-soluble polymer, such as modified starch, gelatin, polyvinyl alcohol, and combinations comprising one or more of the foregoing binders. Another suitable binder includes hydroxyproply cellulose alone or in combination with another binder. Examples of suitable fillers include lactose, microcrystalline cellulose, and combinations comprising one or more the foregoing fillers. An example of a glidant is colloidal silicon dioxide (AEROSIL, Evonik). An example of a suitable plasticizer is triethyl citrate, propylene glycol, or polyethylene glycol.

In some embodiments, the extended release multi-particulates have a particle size (e.g., d₅₀) of less than 500 µm, less than 450 µm, less than 400 µm, less than 350 µm, less than 300 µm, less than 250 µm, less than 200 µm, about 450 µm to about 1 µm, about 450 µm to about 10 µm, about 450 µm to about 20 µm, about 450 µm to about 50 µm, about 450 µm to about 100 µm, about 400 µm to about 1 µm, about 400 µm to about 10 µm, about 400 µm to about 20 µm, about 400 µm to about 50 µm, about 400 µm to about 100 µm, or any combination thereof.

In some embodiments, the extended-release multi-particulates can further comprise a pH-controlling agent. The amount of a pH-controlling agent is sufficient to provide pH independent release of droxidopa from the multi-particulate.

Examples of a pH controlling agent (term used interchangeably wiht a pH modifier or a buffering agent) include pharmaceutically acceptable buffering systems, acids having suitable pKₐ and/or their salts, for example citric acid, citrate salts, tartaric acid, tartarate salts, succinic acid, succinate salts, acetic acid, acetate salts, fumaric acid, and fumarate salts. Inorganic acids can also be used, including hydrochloric or sulfuric acid.

In some embodiments, the pH modifier is not in direct contact with droxidopa, or a pharmaceutically acceptable salt thereof of the multi-particulate. In some embodiments, the liquid vehicle comprises a pH modifier (e.g., monosodium citrate or sodium bitartarate) 0.1% w/v to 1.0% w/v of the liquid dosage (e.g., suspension) volume.

In some embodiments, a multi-particulate disclosed herein is combined with a liquid vehicle. In some embodiments, the liquid vehicle comprises a pH modifier. In some embodiments, the pH modifier is chosen such that when supplied as ready-to-use suspension or when the multi-particulates are reconstituted or suspended with a liquid vehicle, such as water, the pH modifier can maintain the pH of the reconstituted liquid dosage form, e.g., at a pH of < 7.0, for example, from about 6.0 to about 7.0, from about 2.0 to about 5.0, from about 2.0 to about 4.5, from about 2.0 to about 4.0, from about 2.5 to about 5.0, from about 2.5 to about 4.5, from about 2.5 to about 4.0, from about 3.0 to about 5.0, from about 3.5 to about 4.5, or from about 3.5 to about 4.0. In some embodiments, the liquid dosage form has a pH of about 4.0.

In other embodiments, the liquid dosage form comprising a multi-particulate has a pH above 7.0, such as about 7.0 to about 8.0, or about 7.0 to about 9.0. In some liquid dosage forms, droxidopa is protected from degradation by a protective layer that prevents exposure of the drug to the high pH.

In some embodiments, the multi-particulate does not comprise a pH-controlling agent.

The multi-particulate can also be coated with a protection layer. In some embodiments, the protection layer prevents drug release from the multi-particulates reconstituted or suspended into a liquid dosage form or into the food on which it is sprinkled. Examples of a protection layer include, for example, a moisture protection layer, a light protection layer, an integrity protection layer to prevent friable dosage forms from crumbling, a taste masking protective layer, and protective layers that increase ease of swallowing. Examples of materials that may be used in a moisture protection layer include a cationic methacrylate copolymer, for example EUDRAGIT E100, a cationic copolymer based on dimethylaminoethyl methacrylate, butyl methacrylate, methyl methacrylate, or a combination of the foregoing. Polyvinyl alcohol, ethyl cellulose, and hydroxypropyl methyl cellulose may also be used in moisture protection layers and other types of protection layers. Unless clearly indicated by the context of this disclosure, protection layers do not significantly affect drug release profiles and are not controlled release coatings.

The extended-release multi-particulate comprises a release-controlling agent. The release-controlling agent can comprise a polymer, a non-polymeric material, or a combination thereof.

Examples of a polymeric release controlling agent include shellacs, ethylcellulose, cellulose acetate phthalate, acrylic resins, methacrylate hydrogels, methylmethacrylate, polymethacrylate, polylactic acid, polyvinyl acetate, polyvinyl chloride, polymethacrylate, hydroxypropylmethylcellulose, polyethylene glycols, carboxymethylcellulose, sodium carboxymethylcellulose, and combinations thereof.

The non-polymeric release-controlling agent can comprise a wax, a lipophilic compound, or a combination thereof.

The wax can be, for example, an amorphous wax, an anionic wax, an anionic emulsifying wax, a bleached wax, a carnauba wax, a cetyl esters wax, a beeswax, a castor wax, a cationic emulsifying wax, a cetrimide emulsifying wax, an emulsifying wax, a glyceryl behenate, a microcrystalline wax, a nonionic wax, a nonionic emulsifying wax, a paraffin, a petroleum wax, a spermaceti wax, a white wax, a yellow wax, and combinations comprising one or more of the foregoing waxes. These and other suitable waxes are known to those of skill in the art. A cetyl esters wax, for example, preferably has a molecular weight of about 470 to about 490 and is a mixture containing primarily esters of saturated fatty alcohols and saturated fatty acids. The wax can comprise a carnauba wax, glyceryl behenates (e.g., glyceryl dibehenate), castor wax, and combinations comprising one or more of the foregoing waxes.

The wax material can be used at about 10 wt % to about 70 wt%, 13 wt % to about 55 wt%, about 16 wt% to about 40 wt%, about 20 wt% to about 36 wt%, about 24 wt% to about 33 wt% of the total weight of the multi-particulate. When a combination of wax is used, e.g., carnauba wax and glyceryl behenate, the component waxes can be used in a suitable ratio. Certain formulations include the wax material component from 100 to about 85 parts carnauba wax and from 0 to about 15 parts glyceryl behenate. In formulations that have a combination of carnauba wax and castor wax, for example, the wax component may have about 100 to about 85 parts carnauba wax and 0 to about 15 parts castor wax. When carnauba wax, glyceryl behenate and castor wax are present, the carnauba wax can comprise at least about 85 wt% of the waxy material and the balance of the waxy material is made up of a combination of glyceryl behenate and castor wax, in a suitable relative proportion.

The lipophilic compound can be, for example, a fatty acid, a fatty acid soap; a fully or partially hydrogenated vegetable oil or fat; or a mono-, di-, or triacylglceride. The fatty acids and fatty acid soaps can be those that are generally used in the pharmaceutical industry as tableting lubricants, such as, for example, solid fatty acids (for example fatty acids having from about 16 to about 22 carbon atoms), and the alkaline earth metal salts thereof, particularly the magnesium and calcium salts, and combinations comprising one or more of the foregoing fatty acids. The fatty acid can be, for example, stearic acid. Examples of the fully or partially hydrogenated vegetable oil or fat include hydrogenated palm fat and oil, hydrogenated castor oil, hydrogenated rape oil, hydrogenated cottonseed oil, hydrogenated soybean oil, and hardened soybean oil. Examples of a mono-, di-, or triglyceride include glyceryl monolaurate; glyceryl dilaurate; glyceryl monomyristate; glyceryl dimyristate; glyceryl monopalmitate; glyceryl dipalmitate; glyceryl monostearate; glyceryl distearate; glyceryl monooleate; glyceryl dioleate; glyceryl monolinoleate; glyceryl dilinoleate; glyceryl monoarachidate; glyceryl diarachidate; and glyceryl dibehenate, glycerol behenate, glyceryl palmitostearate, trilaurin, trimyristan, tripalmitin, tristearin, and tribehenin. The lipophilic compound can be used in amounts of up to about 70 wt% of the total weight of the multi-particulate, or about 2.5 wt% to about 55 wt%, or about 2.7 wt% to about 40 wt%, or from about 3 wt% to about 36 wt%, or from about 3.5 wt% to about 33 wt% of the total weight of the multi-particulate.

In some embodiments, the oral dosage form disclosed herein comprises immediate release multi-particulates and extended release multi-particulates. In some embodiments, the oral dosage form disclosed herein comprises fast release multi-particulates and extended release multi-particulates. In certain embodiments, the oral dosage form comprises two or more types of extended-release multi-particulates comprising droxidopa, or a pharmaceutically acceptable salt thereof. Each type of extended-release multi-particulate can have a different extended release profile for droxidopa, or the pharmaceutically acceptable salt thereof.

In certain embodiments, the droxidopa, or the pharmaceutically acceptable salt thereof, is released from a first multi-particulate at a pH of about 1.0 to about 3.5 (e.g., about pH 1.2) and the droxidopa, or the pharmaceutically acceptable salt thereof, is released from a second multiparticulate at a pH of 5.5. In some embodiments, the first multi-particulate and the second multi-particulate are pellets, granulates, or mini-tablets. In a further embodiment, the composition can comprise an immediate release multi-particulate comprising droxidopa, or a pharmaceutically acceptable salt thereof.

The composition or suspension disclosed herein comprises (i) a first multi-particulate comprising droxidopa, or a pharmaceutically acceptable salt thereof, and optionally a first release controlling agent; and (ii) a second multi-particulate comprising droxidopa, or a pharmaceutically acceptable salt thereof, and a second release controlling agent. In some embodiments, the release controlling agent (e.g., the first and/or second release controlling agent) is non-polymeric release controlling agent. In some embodiments, the release controlling agent (e.g., the first and/or second release controlling agent) comprises a wax or a wax/lipid matrix. In some embodiments, the compostion or suspension further comprises a liquid vehicle.

In some embodiments, the composition or suspension disclosed herein comprises (i) a first multi-particulate comprising droxidopa, or a pharmaceutically acceptable salt thereof, in an immediate release form, and a moisture protective layer (e.g., EUDRAGIT E100); and (ii) a second multi-particulate comprising droxidopa, or a pharmaceutically acceptable salt thereof, and a release controlling agent. In one embodiment, the second multi-particulate comprises a wax/lipid matrix (e.g., hydrogenated castor oil). The second multi-particulate is coated with an enteric protective layer (e.g., EUDRAGIT 30-D55).

In some embodiments, the composition or suspension disclosed herein comprises (i) a first multi-particulate comprising droxidopa, or a pharmaceutically acceptable salt thereof, in fast release form, and a moisture protective layer (e.g., EUDRAGIT E100); and (ii) a second multiparticulate comprising droxidopa, or a pharmaceutically acceptable salt thereof, and a release controlling agent. In one embodiment, the second multi-particulate comprises a wax/lipid matrix (e.g., hydrogenated castor oil). The second multi-particulate is coated with an enteric protective layer (e.g., EUDRAGIT 30-D55).

In some embodiments, the composition or suspension disclosed herein comprises (i) a first multi-particulate comprising droxidopa, or a pharmaceutically acceptable salt thereof, and a first release controlling agent; and (ii) a second multi-particulate comprising droxidopa, or a pharmaceutically acceptable salt thereof, and a second release controlling agent. In one embodiment, the first multi-particulate comprises a wax or a wax/lipid matrix (e.g., glyceryl dibehenate). In one embodiment, the first multi-particulate does not comprise a protective layer. In one embodiment, the second multi-particulate comprises a wax or a wax/lipid matrix (e.g., hydrogenated castor oil). The second multi-particulate is coated with an enteric protective layer (e.g., EUDRAGIT 30-D55).

In some embodiments, the composition or suspension disclosed herein comprises (i) a first multi-particulate comprising droxidopa, or a pharmaceutically acceptable salt thereof, and a first non-polymeric release controlling agent (e.g., a wax); and (ii) a second multi-particulate comprising droxidopa, or a pharmaceutically acceptable salt thereof, and a second non-polymeric release controlling agent (e.g., a wax). In some embodiments, the composition or suspension further comprises an aqueous liquid vehice comprising a pH modifier (e.g., monosodium citrate and/or sodium bitartarate) and a suspending agent (e.g., xantham gum). In some embodiments, the first multi-particulate comprises 200 mg to 400 mg (e.g., about 300 mg) of droxidopa and the second multi-particulate comprises 500 mg to 700 mg (e.g., about 600 mg) of droxidopa. In some embodiments, the wax is glyceryl dibehanate. The second multiparticulate is coated with an enteric protective layer (e.g. EUDRAGIT 30-D55). In some embodiments, the liquid vehicle further comprises a preservative, a filler, a sweetener, a flavoring agent, a coloring agent, a pH modifier, or any combination thereof. In some embodiments, the aqueous liquid vehice has a pH of less than 7, e.g., about 3 to 5, preferably about 4.

In some embodiments, the first and/or second multi-particulate can comprise a pH controlling agent, such as a citrate, a tartrate, or combinations thereof (e.g., sodium bitartarate, monosodium citrate).

In some embodiments, the multi-particulate are pellets. In some embodiments, the pellets are formed by direct pelletization. In some embodiments, the pellets have a particle size of less than 400 µm.

In some embodiments, the multi-particulate is a pulsatile release multi-particulate. The composition or suspension disclosed herein can comprises one or more types of pulsetile release multi-particulates comprising droxidopa, or a pharmaceutically acceptable salt thereof. Each type of pulsetile release multi-particulates releases the drug at a different time. In one embodiment, the composition or suspension comprises two types of pulsetile release multiparticulates. In another embodiment, the composition or suspension comprises three types of pulsetile release multi-particulates.

Pulsatile release multi-particulates for each pulse can release the same or different amounts of the active agent. In some embodiments, pulsatile release multi-particulates for each pulse releases the same amount of the active agent. In some embodiments, pulsatile release multi-particulates for each pulse releases a different amount of the active agent.

Pulsatile release multi-particulates for each pulse can have the same or different extended release periods. In some embodiments, pulsatile release multi-particulates for each pulse have the same extended release period, such as about 4 hours. In some embodiments, pulsatile release multi-particulates for each pulse have the same or a different extended release period, e.g., an extended release period of about 1 hour, 2 hours, 3 hours, 4 hours, 5 hours, 6 hours, 7 hours, 8 hours, or any combination thereof.

### Dosage

A daily dosage amount of droxidopa, or a pharmaceutically acceptable salt thereof, in the oral dosage form is about 100 mg to about 1800 mg or about 100 mg to about 3000 mg. In some embodiments, the composition comprising droxidopa, or a pharmaceutically acceptable salt thereof, is administered at a dose of about 100 mg to about 3000 mg, about 200 mg to about 3000 mg, about 300 mg to about 3000 mg, about 400 mg to about 3000 mg, about 500 mg to about 3000 mg, about 600 mg to about 3000 mg, about 700 mg to about 3000 mg, about 800 mg to about 3000 mg, about 900 mg to about 3000 mg, about 1000 mg to about 3000 mg, about 1500 mg to about 3000 mg, about 2000 mg to about 3000 mg, about 100 mg to about 1800 mg, about 200 mg to about 1800 mg, about 300 mg to about 1800 mg, about 400 mg to about 1800 mg, about 500 mg to about 1800 mg, about 600 mg to about 1800 mg, about 700 mg to about 1800 mg, about 800 mg to about 1800 mg, about 900 mg to about 1800 mg, about 500 mg to about 1200 mg, about 600 mg to about 1000 mg, or about 800 mg to about 1000 mg once or twice daily. In some embodiments, the composition comprising droxidopa, or a pharmaceutically acceptable salt thereof, is administered at a dose of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, about 2800 mg, about 2900 mg, or about 3000 mg once or twice daily.

In some embodiments, the composition or suspension disclosed herein provides for a daily dosage of about 330 mg to about 500 mg, about 660 mg to about 1000 mg, about 495 mg to about 1500 mg, about 990 mg to about 1500 mg, about 990 mg to about 3000 mg, or about 100 mg to about 3000 mg of droxidopa, or a pharmaceutically acceptable salt thereof.

In some embodiments, the composition or suspension disclosed herein provides for a one time daily dosage of about about 990 mg to about 3000 mg of droxidopa, or a pharmaceutically acceptable salt thereof. In some embodiments, the composition or suspension disclosed herein provides for a twice daily dosage of about about 495 mg to about 1500 mg of droxidopa, or a pharmaceutically acceptable salt thereof.

The extended release oral dosage form can provide a droxidopa plasma level in a subject of about 0.5 µg/mL to 5 µg/mL for a duration of about 4 to 24 hours or about 4 to 16 hours after oral administration of the composition to the subject.

The composition can be packaged to provide a single dose or multiple doses, for example 2, 3, 4, 5, 6, 7, 8, or more doses. The composition can be packaged to provide a single daily dosage amount or the daily dosage amount for multiple days, for example at least 3, 4, 5, 6, or 7 days.

In certain embodiments, the composition can be in the form of a capsule filled with the extended-release multi-particulate. In yet other embodiments, the composition is in the form of a compressed tablet comprising the multiparticulate. Alternatively, the composition can be in the form of a sealed package, such as a sachet, a two compartment sachet or the cap of a bottle, into which the multi-particulate has been filled. In another embodiment, the composition can be in the form of a suspension comprising one or more types of multi-particulates and a suspending vehicle.

### Solution, Suspension, or Emulsion

In some embodiments, a solid pharmaceutical composition disclosed herein (e.g., a multi-particulate) is suitable for being reconstituted or suspended as a liquid dosage form (e.g., a suspension) or to be sprinkled on food or in a drink.

Some aspects of the application are directed to an extended-release suspension for oral administration comprising (a) first and second multi-particulates as defined in the claims, and (b) a liquid vehicle.

In another aspect, the application is directed to a solid composition comprising an extended-release multi-particulate of the application, which is suitable for reconstitution or suspension in a liquid vehicle. In some embodiments, the solid composition is packaged in a sachet, a bottle or a packet. In some embodiments, the solid composition is combined with a liquid vehicle, e.g., water, for oral administration.

The liquid vehicle can be an aqueous vehicle or a nonaqueous vehicle. Examples of aqueous vehicles include water and a buffered solution. Examples of nonaqueous vehicles include glycerin and propylene glycol.

The liquid vehicle can comprise excipients such as a stabilizer, a suspending agent, a flavoring agent, or a combination thereof. The liquid vehicle can be a suspending vehicle. In some embodiments, the suspending vehicle can comprise excipients such as suspending agent, a filler, a sweetener, a viscosity modifier, a flowing aid, a pH modifier, a preservative, a surfactant, a flavoring agent, a coloring agent, or a combination thereof.

In some embodiments, the liquid vehicle comprises a suspending agent. In some embodiments, the suspending agent is 0.1% w/v to 1.0% w/v of the liquid vehicle (e.g., suspension) volume. In some embodiments, the suspending agent is xanthan gum, guar gum, sodium carboxymethycellulose, hydroxypropyl cellulose, hypromellose, or any combination thereof.

In some embodiments, the liquid vehicle comprises a preservative. In some embodiments, the preservative is up to 100 mg per unit. In some embodiments, the preservative is 0.2% w/v to 1% w/v of the liquid vehicle (e.g., suspension) volume. In some embodiments, the preservative is sodium benzoate, potassium sorbate, sodium propionate, methylparaben, propylparaben, benzoic acid, sorbic acid, boric acid, or any combination thereof.

In some embodiments, the liquid vehicle, solution, suspension, or emulsion has a pH < 7.0. In some embodiments, the pH is from 7.0 to 8.0, or from 7.0 to 9.0. In some embodiments, the pH is less than 5.0. In some embodiments, the pH is from about 2.0 to about 5.0, or from about 3.0 to about 5.0. In some embodiments, the pH is about 4.0.

In some embodiments, the liquid vehicle has a pH of less than 7.0. In some embodiments, the liquid vehicle includes pH modifier. In some embodiments, the liquid vehicle does not include a pH modifier.

Examples of a pH modifier include pharmaceutically acceptable buffering systems, acids having suitable pKₐ and/or their salts, for example citric acid, citrate salts, tartaric acid, tartarate salts, succinic acid, succinate salts, acetic acid, acetate salts, fumaric acid, and fumarate salts. Inorganic acids can also be used, including hydrochloric or sulfuric acid.

The liquid vehicle can further comprise a polymer that forms an in situ gel in the gastrointestinal tract. The liquid vehicle for reconstituting a solid droxidopa multi-particulate into a liquid dosage form may comprise materials which are in solution or suspension form before administration to the subject, but once administered undergo gelation *in situ* in the body to form a gel from which the drug is released in a sustained and controlled manner. The formation of a gel depends on various factors such as temperature modulation, pH change, and the presence of ions. Examples of polymers that can be used in the liquid vehicle for formation of an *in situ* gel after administration include gellan gum (induced by cations), sodium alginate (induced by cations), xyloglucan (induced by temperature), pectin (induced by calcium cation), chitosan, carbomer(induced by pH), poly(DL-lactic acid), poly(DL-lactide-co-glycolide), and poly-caprolactone.

In some embodiments, the volume of the liquid dosage form (e.g., suspension) to be administered to a subject is about 5 mL to about 100 mL (e.g., about 10 mL to about 100 mL, about 10 mL to about 75 mL, or about 30 mL to about 60 mL). In some embodiments, the liquid dosage form is administered once or twice daily. In some embodiments, a dose of about 100 mg to about 1800 mg or about 100 mg to about 3000 mg of droxidopa, or a pharmaceutically acceptable salt thereof, in a liquid dosage from is administered to a subject in need thereof.

In some embodiments, the liquid composition (e.g., a solution, a suspension, or an emulsion) comprising a multi-particulate comprising droxidopa, or pharmaceutically acceptable salt there, is stable when stored at room temperature or 4 °C for at least 1, 2, 3, 4, 5, 6, 7, 8, 9, 10, 11, 12, 13, 14, 15, 16, 17, 18, 19, 20, or 21 days after reconstitution or suspension of the droxidopa, or the pharmaceutically acceptable salt there, in a liquid vehicle. In some embodiments, the droxidopa, or the pharmaceutically acceptable salt there, present in the liquid vehicle or the suspending vehicle is stable at room temperature or 4 °C for at least 7 days, at least 10 days, at least 14 days, or at least 21 days (e.g., at least one week, at least two weeks, or at least three weeks) after reconstitution or suspension. The stability of droxidopa is measured by the level of the major degradant (i.e., dihydroxybenzaldehyde) and the total degradation. In some embodiments, the droxidopa in the liquid vehicle or the suspending vehicle is stable when the level of dihydroxybenzaldehyde is below 0.1%, 0.05%, 0.04%, 0.03%, 0.02%, or 0.01%. In some embodimnts, the droxidopa in the liquid vehicle or the suspending vehicle is stable when the level of the total degradation is below 1%, 0.5%, 0.4%, 0.3%, 0.2%, or 0.1%.

In certain embodiments, the oral dosage form further comprises droxidopa, or a pharmaceutically acceptable salt thereof, in an immediate release form. The composition or suspension can include ≤ 14% or ≥ 56% of the total amount of droxidopa, or a pharmaceutically acceptable salt thereof, in the dosage form in an immediate release form. In some embodiments, the amount of droxidopa, or a pharmaceutically acceptable salt thereof, in an immediate release form is 1% to 14%, 2% to 14%, 3% to 14%, 4% to 14%, 5% to 14%, 6% to 14%, 7% to 14%, 8% to 14%, 9% to 14%, 10% to 14%, 11% to 14%, 12% to 14%, 1% to 13%, 2% to 13%, 3% to 13%, 4% to 13%, 5% to 13%, 6% to 13%, 7% to 13%, 8% to 13%, 9% to 13%, 10% to 13%, 11% to 13%, 1% to 12%, 2% to 12%, 3% to 12%, 4% to 12%, 5% to 12%, 6% to 12%, 7% to 12%, 8% to 12%, 9% to 12%, 10% to 12%, 1% to 11%, 2% to 11%, 3% to 11%, 4% to 11%, 5% to 11%, 6% to 11%, 7% to 11%, 8% to 11%, 9% to 11%, 1% to 10%, 2% to 10%, 3% to 10%, 4% to 10%, 5% to 10%, 6% to 10%, 7% to 10%, 8% to 10%, less than 14%, less than 12%, less than 10%, less than 8%, less than 6%, less than 5%, less than 4%, less than 3%, less than 2% or less than 1% of the total weight of droxidopa in the composition or suspension.

In some embodiments, the composition or suspension disclosed herein can comprise droxidopa, or a pharmaceutically acceptable salt thereof, in an immediate release form, in an amount of 20% to 40%, 20% to 35%, 20% to 30%, 25% to 40%, 25% to 35%, 25% to 30%, 30% to 40%, 30% to 35%, or 35% to 40% of the total weight of droxidopa in the composition or suspension.

In some embodiments, the composition or suspension disclosed herein can comprise droxidopa, or a pharmaceutically acceptable salt thereof, in a fast release form, in an amount of 20% to 40%, 20% to 35%, 20% to 30%, 25% to 40%, 25% to 35%, 25% to 30%, 30% to 40%, 30% to 35%, or 35% to 40% of the total weight of droxidopa in the composition or suspension.

### Kits

In another aspect, a pharmaceutical kit is disclosed herein. The kit can comprise any of the solid pharmaceutical compositions disclosed herein (e.g., a multi-particulate packaged in a sachet, a bottle or a packet) and a liquid vehicle for reconstituting or suspending the solid composition into an oral liquid dosage form, e.g., a suspension. The liquid vehicle can be a suspending vehicle. In some embodiments, the liquid dosage form produced can have a pH < 7.0. In some embodiments, the liquid dosage form produced can have a pH from 7.0 to 8.0, or from 7.0 to 9.0. In some embodiments, the pH is less than 5.0. In some embodiments, the pH is from about 2.0 to about 5.0, or from about 3.0 to about 5.0.

The kit can be in the form of a capped bottle in which the composition is stored in a sealed compartment within the cap of the bottle and the liquid vehicle is stored in the bottle. In some embodiments, the cap of the bottle stores a solid dosage form (e.g., a multi-particulate) comprising droxidopa, or a pharmaceutically acceptable salt thereof, in a sealed compartment within the cap of the bottle. In some embodiments, the liquid vehicle (i.e., a suspending vehicle) is stored in the bottle. In some embodiments, the sealed compartment within the cap of the bottle is unsealed and the solid dosage form is combined with the liquid vehicle. The solid dosage form comprises two or more types of multi-particulate pellets and the liquid vehicle is a suspending vehicle. In some embodiments, some excipients of the liquid vehicle (e.g., a suspending agent or a gel forming polymer) can be stored in a solid form in the kit.

In some embodiments, the solid dosage form (e.g., a multi-particulate) is stored in a container (e.g., a sachet), and a patient can open the container, mix the solid dosage with water or a beverage, or sprinkle the solid dosage on food, for oral consumption.

In certain aspects of the disclosure, the kit comprises a single dose or multiple doses, for example 2, 3, 4, 5, 6, 7, 8, or more doses. The kit (e.g., a single bottle) can be prepared to provide a single daily dosage amount or the daily dosage amount for multiple days, for example at least 3, 4, 5, 6, or 7 days. In some embodiments, the liquid dosage form is a suspension that is stable at room temperature or 4 °C for at least 7 days, 10 days, 14 days, or 21 days.

### Dissolution

Active agent release from a pharmaceutical formulation can be analyzed in various ways. One exemplary test is in vitro dissolution. A dissolution profile is a plot of the cumulative amount of active agent released from a formulation as a function of time. A dissolution profile can be measured utilizing the Drug Release Test <724>, which incorporates standard test USP 39 (Test <711>). A profile is characterized by the test conditions selected such as, for example, apparatus type, shaft speed, temperature, volume, and pH of the dissolution medium. More than one dissolution profile may be measured. For example, a first dissolution profile can be measured at a pH level approximating that of the stomach, and a second dissolution profile can be measured at a pH level approximating that of one point in the intestine or several pH levels approximating multiple points in the intestine.

For example, for the currently marketed droxidopa dosage forms, the U.S. FDA suggests evaluation of droxidopa release characteristics and dissolution profiles in 900 mL of 0.1 N HCl, equilibrated at 37 °C ± 0.5 °C using a basket method (USP Apparatus 1) at 100 rpm with sampling times of 5, 10, 15, 20, 30 minutes. Other conditions, such as different pH, extended dissolution time for up to 24 hrs or different apparatus may be used as known in the art. Sample aliquots can be taken at different time intervals and analyzed by high performance liquid chromatography.

Dissolution profile can be measured under other conditions, for example: 900 mL of 0.1 N HCl for 0-2 hours and Phosphate Buffer (pH 6.8) for 2-8 hours or 2-14 hours using an USP II (Paddle) apparatus.

### Pharmacokinetics

Alternatively, active agent release from a pharmaceutical formulation can be determined in a pharmacokinetics study. Design of such a pharmacokinetics study is within the skill of practitioners in the art.

The extended release droxidopa dosage forms when orally administered once daily to a subject, provide a droxidopa plasma level in the subject of about 0.5 µg/mL to 5 µg/mL for a duration of about 4 to 24 hour or about 4 to 16 hours after oral administration to the subject.

In some embodiments, the composition or suspension disclosed herein releases substantially all of the active agent in about 6, 7, or 8 hours post administration. In some embodiments, the composition or suspension disclosed herein provides only one Cₘₐₓ and the Cₘₐₓ is at about 1 to 5 hours post administration.

### Compositions for use in a method of treating a subject

**In** another aspect, compositions for use in a method of treating a subject are disclosed. The method can comprise orally administering an effective amount of a droxidopa liquid dosage form disclosed herein or a solid droxidopa composition herein to a subject in need of treatment of a disease or disorder. The disease or disorder can be hypotension (for example neurogenic orthostatic hypotension (nOH) or intradialytic hypotension), a symptom of Parkinson's disease (e.g., orthostatic hypotension, postural instability (US20130197090)), Down's syndrome (WO2010132128), a demyelinating disease (US8580776), Alzheimer's disease (US8580776), an attention deficit disorder (US8383681), hypersomnia (US2012010293), pain associated with fibromyalgia (US8008285), motor paralysis (US5656669), motor aphasia (US5656669), urinary incontinence (US5266596), dementia (US4690949), antidiuresis (US4647587), postural tachycardia syndrome (US20150374691), tauopathy (US2014249180), fatigue (US2013116286), headaches (US2006035976), neurological deficits or neuronal death induced by brain ischemia (US2001007856), intracranial hypertension or cerebral edema (US2001007856), cancer (WO2016062272), a bacterial infection (WO2016123063), to induce or facilitate micturition (WO2015127558), nasal congestion (WO2005084330), or pain (e.g., acute or chronic) (WO2004032844). Preferably the disease or disorder is hypotension or a symptom of Parkinson's disease. The administering can be once or twice daily, and can occur with the subject in a supine position. In some embodiments, the total daily dose of droxidopa administered to the subject is 100 mg to 1800 mg or about 100 mg to about 3000 mg.

The compositions or oral dosage forms of the present disclosure comprising droxidopa, or a pharmaceutically acceptable salt thereof, can be used to treat or reduce the incidence of a disorder including at least one of: orthostatic hypotension; postural orthostatic tachycardia syndrome (POTS); dysautonomia; symptoms of chronic orthostatic hypotension corresponding to autonomic failure associated with Bradbury-Eggleston syndrome, Shy-Drager syndrome, diabetes mellitus disease, and Parkinson's disease; and retrograde ejaculation. In some embodiments, the compositions or oral dosage forms of the present disclosure can be used to treat orthostatic hypotension in a subject suffering from Parkinson's disease. In some embodiments, the oral dosage form, pharmaceutical composition, or formulation of the present disclosure can be used to treat a subject suffering from or at risk of suffering from postural orthostatic tachycardia syndrome (POTS).

In certain aspects, the method is directed to treating a subject suffering from or at risk of suffering from orthostatic hypotension due to autonomic failure comprising administering an effective amount of a composition or oral dosage form disclosed herein to the subject.

In certain aspects, the method is directed to treating a subject having Parkinson's disease or postural orthostatic tachycardia syndrome (POTS) who suffers from or is at risk of suffering from orthostatic hypotension comprising administering an effective amount of a composition or oral dosage form disclosed herein to the subject.

In some embodiments, the subject is 10-50 years old, 10-25 years old, e.g., 13-18 years old, 13-21 years old, or 13-25 years old. In some embodiments, the subject is male or female. In some embodiments, the subject is male. In some embodiments, the subject is female. In some embodiments, the subject is female, aged 13-25 years old and suffers from POTS. In some embodiments, the subject suffers from Parkinson's disease. In some embodiments, the subject suffers from early-onset Parkinson's disease (e.g., is 50 years old or younger). In some embodiments, the subject is older than 50 years.

The method of treatment can include administering a single dose of a composition or oral dosage form disclosed herein to a subject. In certain embodiments, the composition or oral dosage form comprises droxidopa, or a pharmaceutically acceptable salt thereof, in an amount of about 100 mg to about 3000 mg, about 200 mg to about 3000 mg, about 300 mg to about 3000 mg, about 400 mg to about 3000 mg, about 500 mg to about 3000 mg, about 600 mg to about 3000 mg, about 700 mg to about 3000 mg, about 800 mg to about 3000 mg, about 900 mg to about 3000 mg, about 1000 mg to about 3000 mg, about 1500 mg to about 3000 mg, about 2000 mg to about 3000 mg, about 100 mg to about 1800 mg, about 200 mg to about 1800 mg, about 300 mg to about 1800 mg, about 400 mg to about 1800 mg, about 500 mg to about 1800 mg, about 600 mg to about 1800 mg, about 700 mg to about 1800 mg, about 800 mg to about 1800 mg, about 900 mg to about 1800 mg, about 500 mg to about 1200 mg, about 600 mg to about 1000 mg, or about 800 mg to about 1000 mg. In some embodiments, the composition or oral dosage form comprises droxidopa, or a pharmaceutically acceptable salt thereof, in an amount of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, about 2800 mg, about 2900 mg, or about 3000 mg. In some embodiments, the composition or oral dosage form disclosed herein is administered once or twice daily.

In some embodiments, a single dose can be administered to effectively raise blood pressure in a subject and maintain the blood pressure in the subject during an extended release period. A single dose can include the active agent in an amount in milligrams of about 500 mg, about 600 mg, about 700 mg, about 800 mg, about 900 mg, about 1000 mg, about 1100 mg, about 1200 mg, or a range between about 100 mg to about 1800 mg or about 100 mg to about 3000 mg, between about 300 mg to about 1800 mg, between about 500 mg to about 1500 mg, between about 500 mg to about 1200 mg, about 600 to about 1200 mg, or between about 800 mg to about 1000 mg.

In some embodiments, the composition or oral dosage form is a liquid dosage (e.g., a suspension or emulsion) and the dosage can be titrated to an effective level for the subject. A subject can start with an initial dose of the active and the subsequent doses can be adjusted based on the subject's response to the initial dose. Dose titration can be conveniently achieved for the liquid dosage form by adjusting the volume of oral suspension to be administered to the subject. In some embodiments, the volume of the liquid dosage form (e.g., a solution, a suspension, or an emulsion) to be administered to a subject is about 5 mL to about 100 mL (e.g., about 10 mL to about 100 mL, about 10 mL to about 75 mL, about 30 mL to about 60 mL) per dose.

In some embodiments, the methods of the present application are directed to administereing a composition or oral dosage form disclosed herein to a subject to maintain the subject's blood pressure in a clinically acceptable range throughout the day.

The method can include administering the single dose to the subject according to any aspect of the composition or oral dosage form described herein. For example, the method can include administering the single dose to the subject in the form of extended release multiparticulates suspended in a liquid vehicle.

In some embodiments, the method can include administering the single dose to the subject such that the extended release of the active agent into the subject's plasma over the extended release period is characterized by an extended release rate. The method may include administering the single dose to the subject such that greater than about 50% (w/w) of the active agent in the pharmaceutical composition is released to the subject over the extended release period. The extended release period can be, in hours, at least one of, or at least about one of: 4, 5, 6, 7, 8, 9, 10, 11, 12, 13, 14, 15, or 16 hours, e.g., about 12 hours, or a range between any two of the preceding values, for example, of between about 4 hours and about 12 hours, between 6 hours to 12 hours, between 8 hours to 12 hours, between 8 hours to 14 hours, or between 8 hours to 16 hours.

In some embodiments, the composition or oral dosage form described herein is administered once or twice daily to a subject in need thereof. The dose administered can be sufficient to obtain a suitable therapeutic response in the subject.

### Methods of making

A variety of processes can be used to produce the extended release multi-particulates comprising droxidopa and the release controlling agent. Exemplary processes include hot melt extrusion, melt granulation, extrusion spheronization, direct pelletization, spray drying, and spray congealing.

Generally, melt-granulation techniques involve melting a normally solid release controlling agent, e.g. a wax or a lipophilic compound, and incorporating a powdered active agent therein. The release controlling agent can be pre-heated to a molten state and then mixed with the dry pre-blend of the remaining matrix ingredients. Alternatively, the release controlling agent can be mixed with the dry pre-blend and the entire mass is then heated to melt the release controlling agent. The mixing should be sufficient to homogenously disperse the dry pre-blend into the molten binder. The mixture is then allowed to cool and the mixture can be ground or milled and screened to the desired size. The ground or milled granulate may be mixed with an optional lubricant or other processing aid. The processing aid can include, for example, hydrophobic colloidal silicon dioxide (such as CAB-O-SIL^{®} M5). Hydrophobic silicon dioxide may be used in amounts of less than or equal to about 0.5 wt%, but individual formulations can be varied as required. The blend of the granulate and the processing aids, if any, may be optionally compressed into mini-tablets and then optionally coated.

The preparation of a suitable melt-extruded matrix according to the present invention may, for example, include the steps of blending the active agent, together with a release-controlling agent and optionally a binder material to obtain a homogeneous mixture. The homogeneous mixture is then heated to a temperature sufficient to at least soften the mixture sufficiently to extrude the same. The resulting homogeneous mixture is then extruded, e.g., using a twin-screw extruder, to form strands. The extrudate is preferably cooled and cut into multiparticulates by any means known in the art. The strands are cooled and cut into multiparticulates. The multiparticulates are then divided into unit doses. The extrudate preferably has a diameter of from about 0.1 to about 5 mm and provides controlled release of the therapeutically active agent for a time period of from about 4 to about 24 hours or about 4 to about 16 hours.

Extrusion-spheronization typically begins with damp wet mass which can be extruded with a twin screw extruder in to wet extrudes. The wet extrudes are spheronized in a marumerizer at a predetermined speed. The extruded cylindrically shaped extrudates are gradually transformed into spherical shapes.

There are few techniques available that can be used to directly form pellets. One of the examples of these techniques is rotor granulation. With this advanced technology, pellets with spherical shape, smooth surface and uniform particle size are generated. Physical properties of the pellets can be controlled through ingredients' properties and process parameters.

Spray drying and spray congealing, known as globulation processes, involve atomization of hot melts, solutions, or suspensions to generate spherical particles or pellets. During spray drying, drug entities in solution or suspension is sprayed, with or without excipients, into a hot air stream to generate dry and highly spherical particles. As the atomized droplets come in contact with hot air, evaporation of the application medium is initiated. This drying process continues through a series of stages where the viscosity of the droplets constantly increases until finally almost the entire application medium is driven off and solid particles are formed. Generally, spray-dried pellets tend to be porous.

During spray congealing, a drug substance is allowed to melt, disperse, or dissolve in hot melts of waxes, fatty acids, etc., and sprayed into an air chamber where the temperature is below the melting temperatures of the formulation components, to provide under appropriate processing conditions spherical congealed pellets.

The multiparticulates can optionally be coated with a protection layer, such as a moisture protection layer. The terms "protection layer" and "protective layer" are used interchangeably herein. The most common method used for the application of coating onto multiparticulates is air suspension coating. Other methods include compression coating, solvent evaporation, coacervation, and interfacial complexation.

In some embodiments, the multi-particulate of the disclosure is pepared by direct pelletization, e.g., using rotor granulation. Characteristics of the pellets formed from the direct pelletization process included one or more of spherical shape, smooth surfaces, broad drug load (<1-90%), high density, and/or uniform and small particle size (e.g., less than 400 µm).

In another aspect, a method of making an oral liquid dosage form is disclosed. The method comprises mixing any of the solid droxidopa compositions disclosed herein with a liquid vehicle to produce a liquid dosage form. The liquid dosage form can be a suspension or an emulsion. The liquid dosage form can have a pH < 7.0, < 6.5, < 6.0, < 5.5, or < 5.0. The method can further comprise breaking a seal of a compartment in a bottle in which the solid droxidopa composition is stored to release the composition into the liquid vehicle.

### Definitions

In this specification and claims that follow, references will be made to a number of terms which shall be defined to have the following meanings.

The terms "a" and "an" do not denote a limitation of quantity, but rather denote the presence of at least one of the referenced item. The term "or" means "and/or". The terms "comprising", "having", "including", and "containing" are to be construed as open-ended terms (i.e., meaning "including, but not limited to").

A "dosage form" means a unit of administration of an active agent. Examples of dosage forms include tablets, mini-tablets, capsules, suspensions, solutions, liquids, emulsions, bottles, sachets, or packets, and the like.

An "active agent" means a compound, element, or mixture that when administered to a patient, alone or in combination with another compound, element, or mixture, confers, directly or indirectly, a physiological effect on the patient. The indirect physiological effect may occur via a metabolite or other indirect mechanism. In some embodiments, the active agent is droxidopa.

"Dosing regimen" means the dose of an active agent taken at a first time by a patient and the interval (time or symptomatic) at which any subsequent doses of the active agent are taken by the patient. The additional doses of the active agent can be different from the dose taken at the first time.

A "dose" means the measured quantity of an active agent to be taken at one time by a patient.

"Droxidopa" as used herein refers to droxidopa or a pharmaceutically acceptable salt thereof.

"Droxidopa therapy" refers to medical treatment of a symptom, disorder, or condition by administration of droxidopa. Droxidopa therapy can be considered optimal when effective plasma levels are reached when required. In addition, peak plasma values (Cₘₐₓ) should be as low as possible while still maintaining an effective plasma level throughout the day so as to reduce the incidence and severity of possible side effects.

The term "effective amount" or "therapeutically effective amount" means an amount effective, when administered to a patient, to provide any therapeutic benefit. A therapeutic benefit may be an amelioration of symptoms, e.g., an amount effective to decrease the symptoms of acute gouty arthritis, for example pain associated with an attack of acute gouty arthritis. The amount that is "effective" will vary from subject to subject, depending on the age and general condition of the individual, the particular active agent, and the like. Thus, it is not always possible to specify an exact "effective amount." However, an appropriate "effective" amount in any individual case may be determined by one of ordinary skill in the art using routine experimentation. In certain circumstances a patient may not present symptoms of a condition for which the patient is being treated. A therapeutically effective amount of an active agent may also be an amount sufficient to provide a significant positive effect on any indicium of a disease, disorder, or condition, e.g. an amount sufficient to significantly reduce the severity of hypotension. A significant effect on an indicium of a disease, disorder, or condition is statistically significant in a standard parametric test of statistical significance, for example Student's T-test, where p ≤ 0.05.

In some embodiments, an effective amount or therapeutically effective amount of droxidopa can be an amount of about 100 mg per day to about 1800 mg or about 100 mg to about 3000 mg per day, about 300 mg to about 1800 mg per day, or about 500 mg to about 1800 mg per day.

"Efficacy" means the ability of an active agent administered to a patient to produce a therapeutic effect in the patient.

A "liquid vehicle" as used herein means a solution or a suspension that can be combined with a solid dosage form comprising an active agent for delivery of the active agent to a subject. The liquid vehicle is a suspending vehicle. In some embodiments, the liquid vehicle can be an aqueous vehicle or a nonaqueous vehicle. Examples of aqueous vehicles include, e.g., water and a buffered solution. Examples of nonaqueous vehicles include, e.g., mixture of alcohol with aqueous vehicle.

An "oral dosage form" means a unit dosage form for oral administration.

A "patient" means a human or non-human animal in need of medical treatment. Medical treatment can include treatment of an existing condition, such as a disease or disorder, prophylactic or preventative treatment, or diagnostic treatment. In some embodiments the patient is a human patient.

As used herein, the terms "pH controlling agent," "pH modifier," and "buffering agent" are used interchangeably, unless indicated otherwise. Examples of a pH controlling agent, a pH modifier, or a buffering agent include pharmaceutically acceptable buffering systems, acids having suitable pKₐ and/or their salts, for example citric acid, citrate salts, tartaric acid, tartarate salts, succinic acid, succinate salts, acetic acid, acetate salts, fumaric acid, and fumarate salts. Inorganic acids can also be used, including hydrochloric or sulfuric acid.

"Pharmaceutically acceptable" means that which is generally safe, non-toxic and neither biologically nor otherwise undesirable and includes that which is acceptable for veterinary use as well as human pharmaceutical use.

"Pharmaceutically acceptable salts" includes derivatives of droxidopa, wherein the droxidopa is modified by making acid or base addition salts thereof, and further refers to pharmaceutically acceptable solvates, including hydrates, and co-crystals of such compounds and such salts. Examples of pharmaceutically acceptable salts include, but are not limited to, mineral or organic acid addition salts of basic residues such as amines; alkali or organic addition salts of acidic residues; and the like, and combinations comprising one or more of the foregoing salts. The pharmaceutically acceptable salts include non-toxic salts and the quaternary ammonium salts of the droxidopa. For example, non-toxic acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, and combinations comprising one or more of the foregoing salts. Pharmaceutically acceptable organic salts includes salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH2)n-COOH where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparginate, glutamate, and the like; and combinations comprising one or more of the foregoing salts; organic amine salts such as triethylamine salt, pyridine salt, picoline salt, ethanolamine salt, triethanolamine salt, dicyclohexylamine salt, N,N' dibenzylethylenediamine salt, and the like; and amino acid salts such as arginate, asparginate, glutamate, and the like; and combinations comprising one or more of the foregoing salts. All forms of such derivatives of droxidopa are contemplated herein, including all crystalline, amorphous, and polymorph forms.

"Pharmacokinetic parameters" describe the in vivo characteristics of an active agent (or a metabolite or a surrogate marker for the active agent) over time, such as plasma concentration (C), Cₘₐₓ, Cₙ, C₂₄, Tₘₐₓ, and AUC. "Cₘₐₓ" is the measured plasma concentration of the active agent at the point of maximum, or peak, concentration. "Cₘᵢₙ" is the measured plasma concentration of the active agent at the point of minimum concentration. "Cₙ" is the measured plasma concentration of the active agent at about n hours after administration. "C₂₄" is the measured plasma concentration of the active agent at about 24 hours after administration. The term "Tₘₐₓ" refers to the time at which the measured plasma concentration of the active agent is the highest after administration of the active agent. "AUC" is the area under the curve of a graph of the measured plasma concentration of an active agent vs. time, measured from one time point to another time point. For example AUC₀₋ₜ is the area under the curve of plasma concentration versus time from time 0 to time t, where t can be the last time point with measurable plasma concentration for an individual formulation. The AUC_{0-∞} or AUC_{0-INF} is the calculated area under the curve of plasma concentration versus time from time 0 to time infinity.

The term "subject" includes any human or non-human animal. For example, the methods and compositions disclosed herein can be used to treat a subject having Parkinson's disease. In a particular embodiment, the subject is a human.

The terms "treating" and "treatment" mean implementation of therapy with the intention of reducing in severity or frequency symptoms, elimination of symptoms or underlying cause, prevention of or reducing the risk of the occurrence of symptoms or their underlying cause, and improvement or remediation of damage.

The terms "administer," "administering," "administered" or "administration" refer to any manner of providing an active agent (such as, droxidopa or a pharmaceutically acceptable salt thereof) to a subject or patient. Routes of administration can be accomplished through any means known by those skilled in the art. Such means include oral, buccal, intravenous, subcutaneous, intramuscular, transdermal, and inhalation.

The term "fast release" as used herein refers to a pharmaceutical formulation that releases the active agent contained therein relatively fast, e.g., relase of the drug starts within about 15 minutes to 2 hours after the start of an *in vitro* dissolution test. In some embodiments, the fast release pharmaceutical formulation comprises an immediate release delivery system.

The term "immediate-release" refers to a pharmaceutical formulation characterized by conventional or non-modified release of the active agent immediately after drug administration. In some embodiments, immediate release means greater than or equal to about 75% of the active agent is released within two hours of administration, specifically within one hour of administration.

As used herein, the term "extended release" refers to a pharmaceutical formulation that provides for the gradual release of an active agent over an extended period of time. "Extended-release" includes the release of the active agent at such a rate that blood (e.g., plasma) levels are maintained within a therapeutic range for at least about 4 hours, for about 4 to about 24 hours, about 4 to about 16 hours, or about 6 to about 10 hours. The term steady-state means that a plasma level for a given active agent has been achieved and which is maintained with subsequent doses of the drug at a level which is at or above the minimum effective therapeutic level for a given active agent.

The modifier "about" used in connection with a quantity is inclusive of the stated value and has the meaning dictated by the context (e.g., includes the degree of error associated with measurement of the particular quantity). The notation "± 10%" means that the indicated measurement can be from an amount that is minus 10% to an amount that is plus 10% of the stated value.

"Optional" or "optionally" means that the subsequently described event or circumstance can or cannot occur, and that the description includes instances where the event occurs and instances where it does not. Unless defined otherwise, technical and scientific terms used herein have the same meaning as is commonly understood by one of skill in the art to which this invention belongs.

The following example are merely illustrative of the compositions and methods disclosed herein and are not intended to limit the scope hereof.

### EXAMPLES

### Manufacturing Processes for extended release multi-particulates

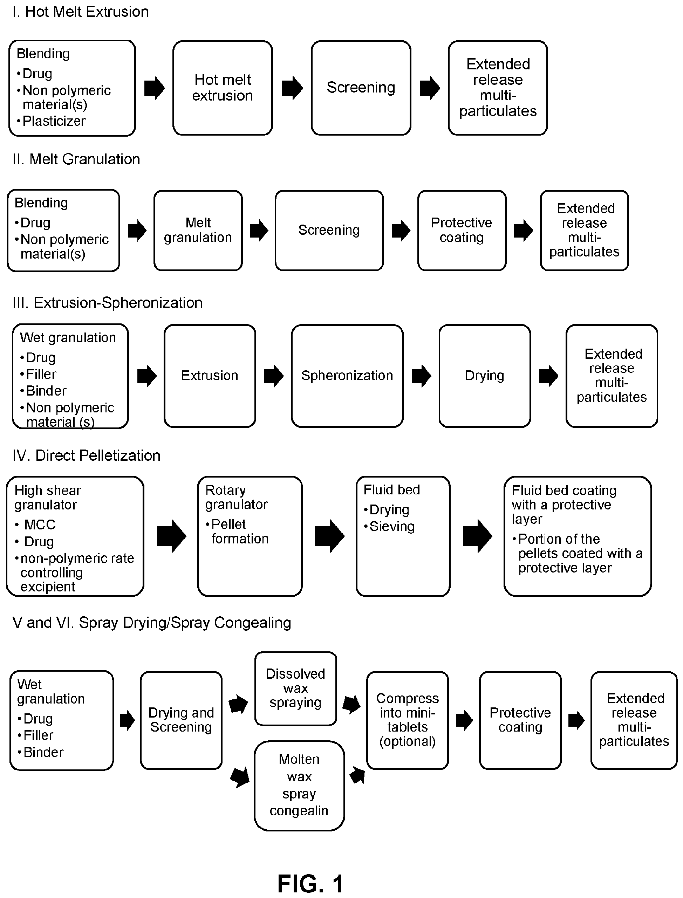

A variety of processes can be used to produce the extended release multi-particulates comprising droxidopa and a non-polymeric release controlling material. Exemplary processes include hot melt extrusion, melt granulation, extrusion spheronization, direct pelletization, spray congealing and wax coating. Schematic flow charts for manufacture of extended release multiparticulates via each of these processes are shown in **FIG. 1****.**

Extended release multi-particulates may also comprise non-release controlling agents such as binders, wetting agents, lubricants, protective coating agents, and surfactants. These excipients are not used to control the drug release, but to improve flowability, stabilize the multiparticulates, minimize premature drug release prior to administration. For example, extended release multi-particulates may be coated with a protective polymer, which is used to minimize premature drug release in the suspension before administration and will dissolve once it is administered.

Only examples 13, formulation I of example 16, and example 7 are according to the claimed invention. The other examples are reference examples.

Example 1. An extended release multi-particulate formulation comprising a lipid matrix with optional moisture protection coating is shown in **Table 1.**

**Table 1**

| **Ingredients** | | **Quantity (mg)** |
|---|---|---|
| Extended release multi-particulates | Droxidopa | 900 |
| | Glyceryl dibehenate | 600 |
| Protective coat (optional) | Eudragit E100 | 70 |
| | Isopropyl alcohol | Quantity sufficient (q.s.) |
| Lubricant | Sodium stearyl fumarate | 30 |
| **Total** | | **1600** |

Example 2. An extended release multi-particulate formulation comprising a lipid matrix is shown in **Table 2.**

**Table 2**

| **Ingredients** | | **Quantity (mg)** |
|---|---|---|
| Extended release multiparticulates | Droxidopa | 1800 |
| | Hydrogenated castor oil | 1000 |
| Lubricant | Sodium stearyl fumarate | 60 |
| **Total** | | **2860** |

Example 3. An extended release multi-particulate formulation comprising droxidopa granules with a lipid coating is shown in **Table 3.**

**Table 3**

| **Ingredients** | | **Quantity (mg)** |
|---|---|---|
| Wet granulation | Droxidopa | 900 |
| | Starch | 200 |
| | Water | q.s. |
| Lipid coating | Hydrogenated castor oil | 500 |
| | Ethanol (optional) | q.s. |
| **Total** | **1600** | |

### Example 4. An extended release multi-particulate formulation comprising droxidopa with a lipophilic coating and protective coating

A protective coating may be applied to the above extended release multi-particulates so that there is no drug release for a period of time, e.g., at least 15 min. An example of the formulation is shown in **Table 4** below. The multi-particulates can be administered as a suspension.

**Table 4**

| **Ingredients** | | **Quantity (mg)** |
|---|---|---|
| Drug substance | Droxidopa | 900 |
| Wax coating | Hydrogenated castor oil | 500 |
| | Ethanol (optional) | q.s. |
| Protective coating | Methacrylic Acid-Ethyl Acrylate Copolymer | 210 |
| **Total** | | **1610** |

### Example 5. Mini-tablets comprising extended release multi-particulates

Extended release granules can be blended with excipients such as diluents, lubricants, and flow aids, and compressed into extended release mini-tablets with a diameter ranging between 1.0-3.0 mm. Any of the extended release multi-particulates disclosed herein or exemplified above can be used. **Table 5** shows an example of extended release multi-particulates.

**Table 5**

| **Ingredients** | **Quantity per single mini-tablet (mg)** | **120 mini-tablets (mg)** |
|---|---|---|
| Extended release multi-particulates | 7.5 | 900 |
| Lactose | 1.2 | 144 |
| Mg stearate | 0.375 | 45 |
| Silicon dioxide | 0.125 | 15 |
| **Total** | **9.2** | **1104** |

### Example 6. Suspensions

Extended release droxidopa multi-particulates are packaged in the cap and a suspending vehicle is filled in the bottle. When opening the bottle, the multi-particulates are released from the cap and fall into the bottle with the suspending vehicle. The suspending vehicle may comprise water, suspending agents, sweeteners, stabilizers, preservatives, coloring agents, surfactants, and flavoring agents. Any of the extended release multi-particulates disclosed herein or exemplified above can be used in these dosage forms. An extended release multi-particulate solid formulation and a suspending vehicle formulation, which can be combined in a bottle to form a suspension, is shown in shown in **Table 6.**

**Table 6**

| **Ingredients** | | **Quantity (mg or mL)** |
|---|---|---|
| Extended release multi-particulates | | 1600 |
| Suspending vehicle | Xanthan gum | 250 |
| | Aspartame | 25 |
| | Polysorbate 80 | 25 |
| | Sodium benzoate | 15 |
| | Cherry flavor | 10 |
| | Coloring agent | 5 |
| | Water | 50 (ml) |

The suspending vehicle may also comprise an alcohol, e.g., ethanol, as shown in **Table 7.**

**Table 7**

| **Ingredients** | | **Quantity (mg or mL)** |
|---|---|---|
| Extended release multi-particulates | | 1600 |
| Suspending vehicle | Xanthan gum | 250 |
| | Aspartame | 25 |
| | Polysorbate 80 | 25 |
| | Sodium benzoate | 15 |
| | Cherry flavor | 10 |
| | Coloring agent | 5 |
| | Water | 49.5 (ml) |
| | Ethanol | 0.5 (ml) |

### Example 7. Ready-to-use suspension dosage form is shown in Table 8.

The extended release multi-particulates are coated to prevent drug release in a ready-to-use suspension. Any of the extended release multi-particulates disclosed herein or exemplified above can be used in this dosage form.

**Table 8**

| **Ingredients** | | **Quantity (mg or mL)** |
|---|---|---|
| Extended release multi-particulates | Droxidopa | 900 |
| | Glyceryl behenate | 600 |
| Protective coat | Eudragit E100 | 150 |
| | Isopropyl alcohol | q.s. |
| Suspending vehicle | Xanthan gum | 150 |
| | Sucralose | 20 |
| | Sodium benzoate | 30 |
| | Cherry flavor | 20 |
| | Coloring agent | 5 |
| | Polysorbate 80 | 15 |
| | Water | 30 (mL) |

Example 8. Extended-release multi-particulates for reconstitution or use as a sprinkle is shown in **Table 9.**

### Solid multi-particulate dosage form

A quantity of extended release multi-particulates suitable for a single dose or for multiple doses are filled into a sachet or packet.

**Table 9**

| **Ingredients** | | **Quantity for a day (mg)** | **Quantity for 7 days (g)** |
|---|---|---|---|
| Extended release multi-particulates | Droxidopa | 900 | 6.30 |
| | Glyceryl behenate | 600 | 4.20 |
| Protective coat | Eudragit E100 | 150 | 1.05 |
| | Isopropyl alcohol | q.s. | q.s. |

### Use as a sprinkle.

The sachet is opened and the extended release multi-particulates are sprinkled on food or a beverage for oral consumption.

### Reconstitution

The contents of a single dose sachet is mixed with a suspending vehicle and reconstituted into a suspension at the time of oral administration.

The contents of a multi-dose sachet is mixed with a suitable volume of suspending vehicle including a preservative and reconstituted into a suspension for oral administration over a period of at least a week.

The suspending vehicle is optionally provided with the sachet as shown in **Table 10.**

**Table 10**

| **Ingredients** | | **Quantity for a day (mg)** | **Quantity for 7 days (g)** |
|---|---|---|---|
| Extended release multi-particulates | Droxidopa | 900 | 6.30 |
| | Glyceryl behenate | 600 | 4.20 |
| Protective coat | Eudragit E100 | 150 | 1.05 |
| | Isopropyl alcohol | q.s. | q.s. |
| Suspending vehicle | Xanthan gum | 150 | 1.05 |
| | Lactose | 900 | 6.30 |
| | Silicon dioxide | 20 | 0.14 |
| | Sucralose | 30 | 0.21 |
| | Sodium benzoate | 10 | 0.07 |
| | Polysorbate | 10 | 0.07 |
| | Peppermint flavor | 30 | 0.21 |
| | **Water** | **30 (mL)** | **210 (ml)** |
| **Total** | | **2800** | **19.6** |

### Example 9. Dosage forms with pH-modifiers

**Table 11** shows droxidopa solubility at room temperature at pH 1.2, 3.0, 4.5, 5.5 and 6.8.

**Table 11**

| **Solution pH** | **Solubility at room temperature (mg/mL)** |
|---|---|
| pH 1.2 | 15.50 |
| pH 3.0 | 2.62 |
| pH 4.5 | 2.35 |
| pH 5.5 | 2.3 |
| pH 6.8 | 2.47 |

Droxidopa is stable in acidic solution, but degrades in neutral or alkaline conditions. Saturated droxidopa in citric acid solution (pH 2.5) or in citrate buffer (pH 4.0) was found to be stable at room temperature over 24 days. When citric acid was present with the drug in solid state, e.g. powder or uncoated granules, droxidopa became unstable under accelerated conditions 70 °C/ 75% RH. When these droxidopa granules/pellets were coated with a protective layer, and blended with citric acid, the drug degradation was reduced.

The effect of pH modifiers or buffering agents on certain liquid dosage forms was tested.

Droxidopa was dissolved in 5 diluents at different pH and stored at room temperature for 24 or 48 hours in sealed glass containers. All solutions were prepared at a nominal concentration of 0.5 mg/ml. Per cent degradation was determined by ultra-performance liquid chromatography (UPLC) on a Waters ACQUITY UPLC with a photodiode array (PDA) detector using a Waters ACQUITY BEH C18 1.7um 2.1x50 mm column. Run conditions for the analyses were as follows:
Mobile phase A: 50mM sodium phosphate pH 3.0
Mobile phase B: Acetonitrile
Detection wavelength: 279 nm
Flow: 0.5 ml/min
Column temperature: 30 C
Injection volume: 2 µl
Gradient (linear transitions) are shown in **Table 12A:**

**Table 12A**

| Time, minutes | % Mobile Phase B |
|---|---|
| Initial | 2 |
| 0.5 | 2 |
| 2 | 30 |
| 2.5 | 30 |
| 2.6 | 2 |
| 3.5 | 2 |

Identification of the known components droxidopa and dihydroxybenzaldehyde was confirmed through retention time and spectral match against pure materials of known identity. Degradation results were quantified as a percent area of degradation peaks versus total area of all degradation/droxidopa peaks. Results for pH 10.0 and pH 12.0 samples were estimated at >90% due to a significant number of peaks present near the retention time of droxidopa.

**Table 13A** below summarizes stability data for droxidopa after exposure in solutions of varying different pH.

**Table 13A**

| pH | Solution diluent | 24-hour Exposure | | 48-hour Exposure | |
|---|---|---|---|---|---|
| | | Major degradant¹, % | Total degradation, % | Major degradant¹, % | Total degradation, % |
| 1.2 | 0.1 N HCl | <0.05 | <0.05 | <0.05 | <0.05 |
| 3 | 50 mM NaH₂PO₄ adjusted to pH 3.0 with Phosphoric acid | <0.05 | <0.05 | <0.05 | <0.05 |
| 7 | 50 mM NaH₂PO₄ adjusted to pH 7.0 with 1N Sodium hydroxide | 0.19 | 0.39 | 0.67 | 1.23 |
| 10 | 25mM HCO₂NH₄ adjusted to pH 10.0 with NH₄OH | 59.64 | >90 | NA | NA |
| 12 | 0.01 N NaOH | 31.92 | >90 | NA | NA |

| | | | | | |
|---|---|---|---|---|---|
| ¹Dihydroxybenzaldehyde | | | | | |

Saturated solutions of droxidopa were prepared in 3 diluents at different pH and stored at room temperature for up to 24 days in sealed containers. All solutions were prepared with an excess of droxidopa by combining 10 mg droxidopa per ml of diluent. Exposed samples were filtered and degradation as a percent of initial droxidopa amount was determined by ultra-performance liquid chromatography (UPLC) on a Waters ACQUITY UPLC with a photodiode array (PDA) detector using a Waters ACQUITY HSS PFP 1.8µm 2.1x100 mm column. Run conditions for the analyses were as follows:
Mobile phase A: 50mM ammonium formate pH 3.0
Mobile phase B: Acetonitrile
Detection wavelength: 279 nm
Flow: 0.5 ml/min
Column temperature: 30 C
Injection volume: 1 µl
Gradient (linear transitions) are shown in **Table 12B:**

**Table 12B**

| Time, minutes | % Mobile Phase B |
|---|---|
| Initial | 0 |
| 1.2 | 0 |
| 5.0 | 20 |
| 6.0 | 20 |
| 6.1 | 0 |
| 8.5 | 0 |

**Table 13B** below summarizes stability data for droxidopa after exposure in saturated solutions.

**Table 13B**

| Solution diluent | 7 day Exposure | | 24 day Exposure | |
|---|---|---|---|---|
| | Major degradant¹, % | Total degradation, % | Major degradant¹, % | Total degradation, % |
| 16 mmol disodium citrate combined with 16mmol citric acid to achieve pH 4.0 | Not detected | 0.0 | 0.01 | 0.1 |
| 16 mmol citric acid | Not detected | 0.0 | 0.01 | 0.1 |
| Deionized water | 0.05 | 0.1 | NA | NA |

| | | | | |
|---|---|---|---|---|
| ¹Dihydroxybenzaldehyde | | | | |

These results show that addition of pH modifiers or buffering agents to the tested liquid dosage forms or one or more components required to reconstitute a solid droxidopa composition to a liquid dosage form can help stabilize the drug.

Examples of suspension formulations with a pH modifier are shown in the **Tables 14 and 15** below.

**Table 14**

| **Ingredients** | | **Quantity (mg or mL)** |
|---|---|---|
| Extended release multi-particulates | | 1600 |
| Suspending vehicle | Xanthan gum | 250 |
| | Aspartame | 25 |
| | Polysorbate 80 | 25 |
| | Sodium benzoate | 15 |
| | Citric acid (pH modifier) | 70 |
| | Cherry flavor | 10 |
| | Coloring agent | 5 |
| | Water | 50 (ml) |

**Table 15**

| **Ingredients** | | **Quantity (mg or mL)** |
|---|---|---|
| Extended release multi-particulates | | 1600 |
| Suspending vehicle | Xanthan gum | 250 |
| | Citric acid (pH modifier) | 50 |
| | Polysorbate 80 | 25 |
| | Sodium benzoate | 20 |
| | Coloring agent | 5 |
| | Water | 40 (ml) |
| | Glycerol | 10 (ml) |

Alternatively, the pH modifier can be included in the extended release multi-particulate. An example extended release multi-particulate form including a pH modifier is shown in the **Table 16** below.

**Table 16**

| **Ingredients** | | **Quantity (mg)** |
|---|---|---|
| Extended release multiparticulates | Droxidopa | 1800 |
| | Hydrogenated castor oil | 1000 |
| pH modifier | Monosodium Citrate | 60 |
| **Total** | | **2860** |

### Example 10. Dosage forms providing in vivo in situ formation of gels

The liquid vehicle for reconstituting a solid droxidopa multi-particulate into a liquid dosage form may comprise materials which are in solution form before administration in the body, but once administered undergo gelation *in situ* to form a gel from which the drug is released in a sustained and controlled manner. The formation of a gel depends on various factors such as temperature modulation, pH change, and the presence of ions. Examples of polymers that can be used in formulations for formation of *in situ* gels after administration include gellan gum (induced by cations), sodium alginate (induced by cations), xyloglucan (induced by temperature), pectin (induced by calcium cation), chitosan, carbomer(induced by pH), poly(DL-lactic acid), poly(DL-lactide-co-glycolide), and poly-caprolactone.

An exemplary formulation of a dosage form providing in situ gel formation and extended release is shown in **Table 17** below.

**Table 17**

| **Ingredients** | | **Quantity (mg or ml)** |
|---|---|---|
| Drug substance | Droxidopa | 900 |
| Liquid vehicle and release controlling agent | Carbomer | 280 |
| | Cherry flavor | 5 |
| | Citric acid | 50 |
| | Polysorbate 80 | 15 |
| | Water | 30 ml |

Furthermore, a pH modifier monosodium citrate was used instead of citric acid and blended with droxidopa granules. Droxidopa was stable for 7 days under accelerated conditions 70 °C/ 75% RH. Another pH modifier sodium bitartrate monohydrate was used the same way and showed similar results. Thus, salt based pH modifiers can be used to improve the stability of the droxidopa.

### Example 11. Ready-to-use suspension with polymer

Ready-to-use suspensions comprising extended release multi-particulates comprising a polymer matrix and a suspending vehicle having a pH < 7.0 are made.

### Extended release multi-particulate formulations comprising a polymer matrix

Extended release multi-particulates are made by dry blending the drug and polymer and then granulated in a fluidized bed granulator using sufficient granulation fluid to produce a wet granulate that is subsequently dried and screened. The multi-particulates optionally include a protection coating. Examples of extended release multi-particulates are show in **Tables 18-20.**

**Table 18**

| **Ingredients** | | **Quantity (mg)** |
|---|---|---|
| Extended release multi-particulates | Droxidopa | 900 |
| | Hypromellose | 600 |
| | Purified Water | q.s. |
| Protective coat (optional) | Eudragit E100 | 70 |
| | Isopropyl alcohol | Quantity sufficient (q.s.) |
| **Total** | | **1570** |

**Table 19**

| **Ingredients** | | **Quantity (mg)** |
|---|---|---|
| Extended release multi-particulates | Droxidopa | 900 |
| | Ethyl cellulose | 80-320 |
| | Microcrystalline Cellulose | 520-280 |
| | Purified Water | q.s. |
| Protective coat (optional) | Eudragit E100 | 70 |
| | Isopropyl alcohol | Quantity sufficient (q.s.) |
| **Total** | | **1570** |

**Table 20**

| **Ingredients** | | **Quantity (mg)** |
|---|---|---|
| Extended release drug loaded granules | Droxidopa | 900 |
| | Polyvinyl alcohol | 320-1000 |
| | Microcrystalline Cellulose | 0-680 |
| | Purified Water | q.s. |
| Protective coat (optional) | Eudragit E100 | 70 |
| | Isopropyl alcohol | Quantity sufficient (q.s.) |
| **Total** | | **2000** |

### Ready-to-use suspension

Any one of above extended release granule formulations, for example the granules including Hypromellose, is mixed with a suspending vehicle comprising a pH modifier (citric acid or monosodium citrate) to form a ready-to-use suspension having a pH < 7.0. An example of a ready-to-use suspension is shown in Table 21.

**Table 21**

| **Ingredients** | | **Quantity (mg or mL)** |
|---|---|---|
| Extended release multiparticulates | Droxidopa | 900 |
| | Hypromellose | 600 |
| Suspending vehicle | Xanthan gum | 250 |
| | Aspartame | 25 |
| | Polysorbate 80 | 25 |
| | Sodium benzoate | 15 |
| | Citric acid | 70 |
| | Cherry flavor | 10 |
| | Coloring agent | 5 |
| | Water | 50 (ml) |

### Example 12. Dissolution

A pH dependence was also observed in drug release from a droxidopa tablet in dissolution tests. The tested tablet formulation is shown in **Table 22** below.

**Table 22**

| **Ingredients** | **Quantity(mg)** |
|---|---|
| Droxidopa | 900.00 |
| Hydrogenated Castor Oil | 400.00 |
| Microcrystalline Cellulose | 186.00 |
| Magnesium Stearate | 14.00 |
| **Total** | **1500.00** |

Droxidopa dissolution from the tablets was tested in 900 mL 0.1N HCl or water in a USP II apparatus (paddle) at 37 °C and 50 rpm. The % drug released over time is shown in **Table 23.**

**Table 23**

| Time (hr) | % drug released (900mL, Paddle, 50 rpm) | |
|---|---|---|
| | 0.1N HCl | Water |
| 0 | 0.0 | 0.0 |
| 1 | 21.8 | 5.9 |
| 2 | 30.5 | 9.4 |
| 4 | 42.2 | 14.2 |
| 6 | 51.4 | 17.9 |
| 8 | 58.2 | 21.2 |
| 12 | 68.2 | 26.9 |
| 18 (Infinity) | 79.3 | 32.9 |

### Example 13. Extended Release Suspension

An extended release droxidopa suspension containing two different types of pellets, pellets I and pellets II, were prepared as shown in **Table 24A.**

Pellets I included a release controlling agent. Upon exposure to pH 1.2 in stomach, Pellets I started releasing the drug over a short period of time, e.g. up to about 2-3 hrs.

Pellets II included a release controlling agent and a protective coating, protecting the drug from releasing in stomach pH 1.2, but allowing release at about pH 5.5. For example, Eudragit L30-D55 was used to protect the drug from releasing in stomach, and can readily dissolve in small intestine at pH 5.5.

Hydroxypropyl cellulose (HPC) was used as a binder and is compatible with the drug. No significant degradation was observed when HPC was used as a dry binder or added as a binder in solution form. The quantity of HPC used is less than 10% w/w, so it does not control drug release in this formulation.

**Table 24A**

| | **Ingredient** | **Functionality** | **Amount in each bottle (mg/bottle)** | **Range (mg/bottle)** |
|---|---|---|---|---|
| Extended Release Pellets I | Droxidopa | Active | 300 | 100-600 |
| | Microcrystalline Cellulose | Filler | 100 | 50-300 |
| | Hydroxypropyl Cellulose | Binder | 23 | 10-36 |
| | Glyceryl dibehenate | Release controlling agent | 100 | 50-200 |
| Extended Release Pellets II with Protective Layer | Droxidopa | Active | 600 | 200-1200 |
| | Microcrystalline Cellulose | Filler | 200 | 100-600 |
| | Hydroxypropyl Cellulose | Binder | 72 | 50-80 |
| | Hydrogenated Castor Oil | Release controlling agent | 400 | 50-400 |
| | Eudragit L30-D55 | Protective layer | 250 | 100-320 |
| | Triethyl citrate | Plasticizer | 50 | 20-70 |
| | Purified Water | Solvent | q.s. | q.s. |
| | **Total** | | **2095** | |
| Suspending Vehicle | Mannitol | Filler & Sweetener | 1500 | 500-2450 |
| | Xanthan Gum | Viscosity modifier | 90 | 10-200 |
| | Colloidal Silicon Dioxide | Flowing aid | 20 | 5-50 |
| | Monosodium citrate | PH modifier | 65 | 30-150 |
| | Sodium lauryl sulfate | Surfactant | 20 | 5-50 |
| | Sodium benzoate | Preservative | 20 | 10-90 |
| | Cherry Flavor | Flavoring | 10 | 5-50 |
| | Coloring agent | Coloring | 10 | 5-50 |
| | Water | Vehicle | 30 mL | 10-60 mL |
| **Total of Suspending Vehicle (without Water)** | | | **1735** | |
| **TOTAL (without Water)** | | | **3830** | |

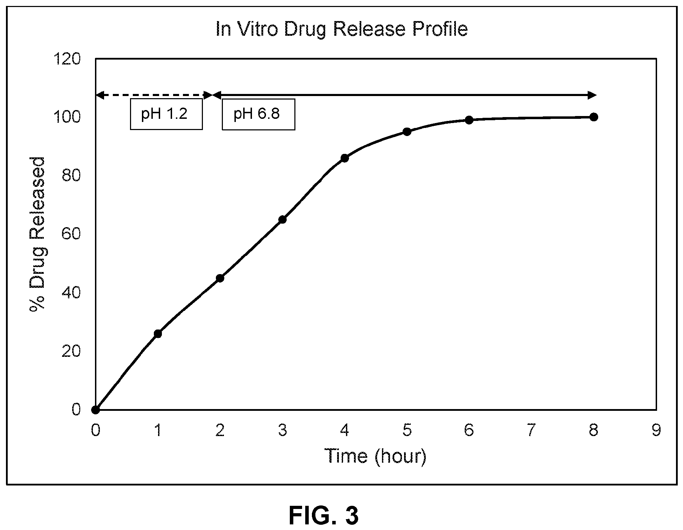

A pH dependence was observed in drug release in a dissolution test performed with USP Apparatus II (Paddles) at 50 rpm in 745 ml of 0.1 N HCl for 2 hours at 37 °C and USP Apparatus II (Paddles) at 50 rpm in 900 ml of pH 6.8 phosphate buffer for 2-8 hours at 37 °C. The dissolution profile (% release over time) is shown in **Table 25** and **FIG. 3****.**

**Table 25**

| ***In vitro* dissolution profile** | | |
|---|---|---|
| **Time (hours)** | **Dissolution media** | **% Drug released *in vitro*** |
| **0** | 0.1 N Hydrochloric acid (HCl) | 0 |
| **1** | | 26 |
| **2** | | 45 |
| **3** | PH 6.8 Phosphate buffered saline | 65 |
| **4** | | 85 |
| **5** | | 95 |
| **6** | | 99 |
| **8** | | 100 |

Alternatively, pellets I were coated with a protective layer which is soluble at pH 5.0 or below and the pH of the suspension can be 7.0 or higher (see **Table 24B**). The protective layer prevents exposure of the drug to higher pH of the suspension and thus degradation. Upon administration, the protective layer dissolves in stomach and drug is released in the similar manner.

**Table 24B**

| | **Ingredient** | **Functionality** | **Amount in each bottle (mg/bottle)** | **Range (mg/bottle)** |
|---|---|---|---|---|
| Pellets I Protective Layer | Droxidopa | Active | 300 | 100-600 |
| | Microcrystalline Cellulose | Filler | 100 | 50-300 |
| | Hydroxypropyl Cellulose | Binder | 23 | 10-36 |
| | Eudragit E100 | Protective layer | 50 | 15-80 |
| | Isopropyl Alcohol | Solvent | q.s. | q.s. |
| Extended Release Pellets II with Protective Layer | Droxidopa | Active | 600 | 200-1200 |
| | Microcrystalline Cellulose | Filler | 200 | 100-600 |
| | Hydroxypropyl Cellulose | Binder | 72 | 50-80 |
| | Hydrogenated Castor Oil | Release controlling agent | 400 | 50-400 |
| | Eudragit L30-D55 | Protective layer | 250 | 100-320 |
| | Triethyl citrate | Plasticizer | 50 | 20-70 |
| | Eudragit E100 | Protective layer | 100 | 30-160 |
| | Isopropyl Alcohol | Solvent | q.s. | q.s. |
| **Total** | | | **2145** | |
| Suspending | Mannitol | Filler & Sweetener | 1500 | 500-2450 |
| Vehicle | Xanthan Gum | Viscosity modifier | 90 | 10-200 |
| | Colloidal Silicon Dioxide | Flowing aid | 20 | 5-50 |
| | Sodium lauryl sulfate | Surfactant | 20 | 5-50 |
| | Sodium benzoate | Preservative | 20 | 10-90 |
| | Cherry Flavor | Flavoring | 10 | 5-50 |
| | Coloring agent | Coloring | 10 | 5-50 |
| | Water | Vehicle | 30 mL | 10-60 mL |
| **Total of Suspending Vehicle (without Water)** | | | **1670** | |
| **TOTAL (without Water)** | | | **3815** | |

No buffering agent was used for pellet I or pellet II. Instead, the drug release for the formulation was controlled by non-polymeric materials.

### Example 14. Extended Release Granules

Extended release granules with 900 mg droxidopa were prepared by melt granulation process and included the ingredients shown in **Table 26.**

**Table 26**

| **Ingredient** | **Amount (mg)** |
|---|---|
| Droxidopa | 900 |
| Microcrystalline cellulose | 300 |
| Hydrogenated castor oil | 800 |
| Total | 2000 |

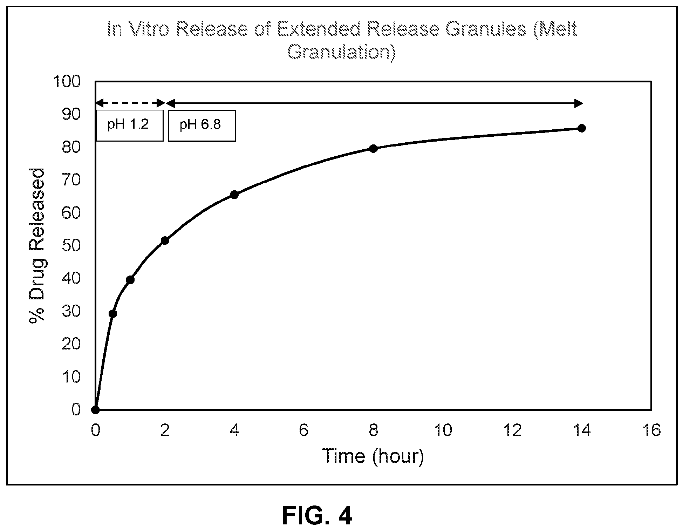

A dissolution test with the extended release granules was performed with USP Apparatus II (Paddles) at 50 rpm in 745 ml of 0.1 N HCl for 2 hours at 37 °C and USP Apparatus II (Paddles) at 50 rpm in 900 ml of pH 6.8 phosphate buffer for 2-14 hours at 37 °C.. The dissolution profile (% release over time) is shown in **FIG. 4****.**

### Example 15. Extended Release Mini-tablets

Extended release mini-tablets with 900 mg droxidopa were prepared by melt granulation and direct compression process and included the ingredients shown in Tables 27 (using wax as the release-controlling agent) and Table 28 (no release controlling agent included).

**Table 27**

| **Ingredient** | **Amount (mg)** |
|---|---|
| Droxidopa | 900 |
| Microcrystalline cellulose | 300 |
| Hydroxyproply cellulose | 60 |
| Hydrogenated castor oil | 90 |
| Total | 1350 |

**Table 28**

| **Ingredient** | **Amount (mg)** |
|---|---|
| Droxidopa | 900 |
| Microcrystalline cellulose | 300 |
| Hydroxyproply cellulose | 60 |
| Sodium stearyl fumarate | 26 |
| Total | 1286 |

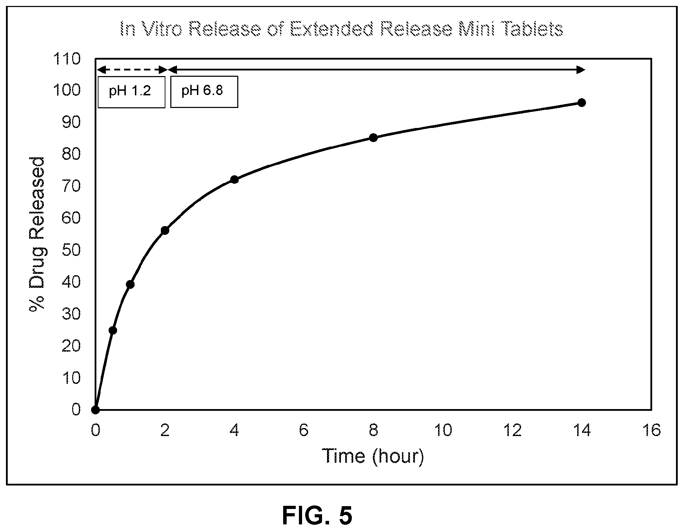

A dissolution test on the mini-tablets was performed with USP Apparatus II (Paddles) at 50 rpm in 745 ml of 0.1 N HCl for 2 hours at 37 °C and USP Apparatus II (Paddles) at 50 rpm in 900 ml of pH 6.8 phosphate buffer for 2-14 hours at 37 °C.. No different in dissolution profile was observed for mini-tablets prepared either by melt granulation or by direct compression. The dissolution profile (% release over time) is shown in **FIG. 5**

### Example 16. Direct Pelletization

Pellets formed by extrusion spheronization process are usually quite limited by the size of the twin-screw screens available. Pellets prepared by extrusion spheronization often have a wide particle size distribution. For an oral extended-release suspension, uniform pellets with a particle size less than 400 µm was desired to avoid a gritty mouth-feel. Further, the four-steps involved in the extrusion spheronization process makes it not economic.

A preferred manufacturing process that could create uniform size pellets with higher drug load was preferred for pellet formation. There were few techniques available that could be used to form such pellets.

An example of a technique for direct pellet formation is rotor granulation. Characteristics of the pellets formed from the direct pelletization process included spherical shape, smooth surfaces, broad drug load (<1-90%), high density, uniform and smaller particle size.

### Formulation I

An example of direct pelletization manufacturing process is described as follows: droxidopa was blended with microcrystalline cellulose and a rate-controlling agent in a high shear granulator, and water was sprayed to form a damp mass. The damp mass was then fed into a rotary granulator where the loose agglomerates were densified and spheronized by an orbital motion while additional water was sprayed. Small and uniform pellets were produced and then dried in a fluid bed processor. A portion of the pellets was further coated in a fluid bed processor with a protective layer. Optionally, the rest of the pellets were coated with a moisture protective layer, which dissolved at pH 1.2. These pellets were blended together to form a uniform blend, which can be filled into a sachet or into a cap of a bottle containing a suspending vehicle in the bottle.

**Table 29**

| | **Ingredients** | **Quantity (mg) per unit** |
|---|---|---|
| **Direct pellet formation - matrix pellets** | Droxidopa | 900 |
| | Microcrystalline cellulose | 1500 |
| | Glyceryl dibehenate | 600 |
| | Purified Water | q.s. |
| Total | | 3000 |
| | | |
| **Pellets I** | **Ingredients** | **Quantity (mg or mL) per unit** |
| | Pellets | 1000 |
| **Pellets II** | Pellets | 2000 |
| | Eudragit L30 D55 | 180 |
| | Propylene glycol | 20 |
| | Purified Water | q.s. |
| | **Total** | **3200** |
| **Suspending vehicle** | Mannitol | 1500 |
| | Monosodium citrate | 140 |
| | Sodium benzoate | 50 |
| | Xanthan gum | 150 |
| | Cherry flavor | 5 |
| | Sodium lauryl sulfate | 10 |
| | Coloring agent | 5 |
| | Water | 50 mL |
| | **Total (without Water)** | **1860** |
| **TOTAL (without Water)** | | **5060** |

### Formulation II:

As an another example of direct pelletization, droxidopa was blended with microcrystalline cellulose in a high shear granulator, and purified water was sprayed onto the blend to form a damp mass. The damp mass was then fed into a rotary granulator where the loose agglomerates were densified and spheronized by an orbital motion. Small uniform pellets were produced and dried in a fluid bed processor. Extended release pellets were produced by further coating these pellets with a non-polymeric material in a fluid bed processor. Spraying molten wax on drug loaded immediate release pellets formed extended release pellets.

**Table 30**

| **Step 1** | | |
|---|---|---|
| | **Ingredients** | **Quantity (mg) per unit** |
| **Direct pellet formation - immediate release pellets** | Droxidopa | 1200 |
| | Microcrystalline cellulose | 1200 |
| | Purified Water | q.s. |

**Step 2**

| | **Ingredients** | **Quantity (mg or mL) per unit** |
|---|---|---|
| **Pellets I** | Immediate release pellets | 800 |
| **Pellets II** | Immediate release pellets | 1600 |
| | Glyceryl dibehenate | 400 |
| | Polyethylene glycol | 20 |
| | Isopropyl Alcohol | q.s. |
| **Suspending vehicle** | Sorbitol | 1500 |
| | Sodium bitartrate | 140 |
| | Guar gum | 150 |
| | Sodium benzoate | 50 |
| | Cherry flavor | 5 |
| | Sodium lauryl sulfate | 10 |
| | Coloring agent | 5 |
| | Water | 50 mL |
| TOTAL (without water) | | 4600 |

### Example 17. Pulsetile Release Formulations

Pulsatile release can be realized by incorporating three different kinds of extended release multi-particulate (e.g., pellets or granules) that each starts releasing the drug at a different time. For example, the first type of multi-particulate (1st pulse) release the drug for a period of about 4 hours. The 2nd type of coated multi-particulate (2nd pulse) will not release any drug until about 4 hours later, but will start to release the drug about 4 hours after the initial administration of the pulsetile release formulation and extends the drug release for 4 hours after the 1st pulse release period. The 3rd type of coated multi-particulate (3rd pulse), will not release the drug until about 8 hours later, but will start to release the drug 8 hours after the initial administration of the pulsetile release formulation and extends the drug relase for 4 hours after the 2nd pulse release period and for a total of 12 hours extended release following the initial administration of the pulsetile release formulation.

Extended release granules can be prepared by wet granulation process followed by a melt granulation process. Drug, a filler, and a binder are blended and granulated in a high shear granulator forming wet granules, which are dried in a fluid bed processor. The granules are mixed with a wax, e.g. hydrogenated castor oil and heated up to the temperature above melting point of the wax, then granulated to form extended release granules. These granules can be coated with different levels of coating which start releasing the drug at different pH.

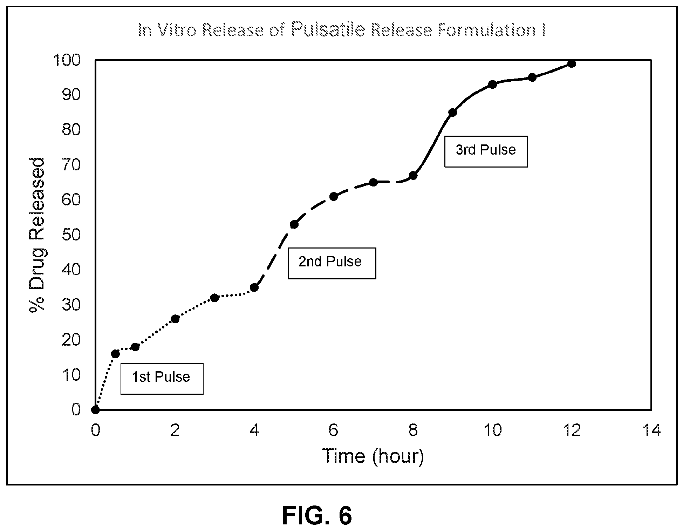

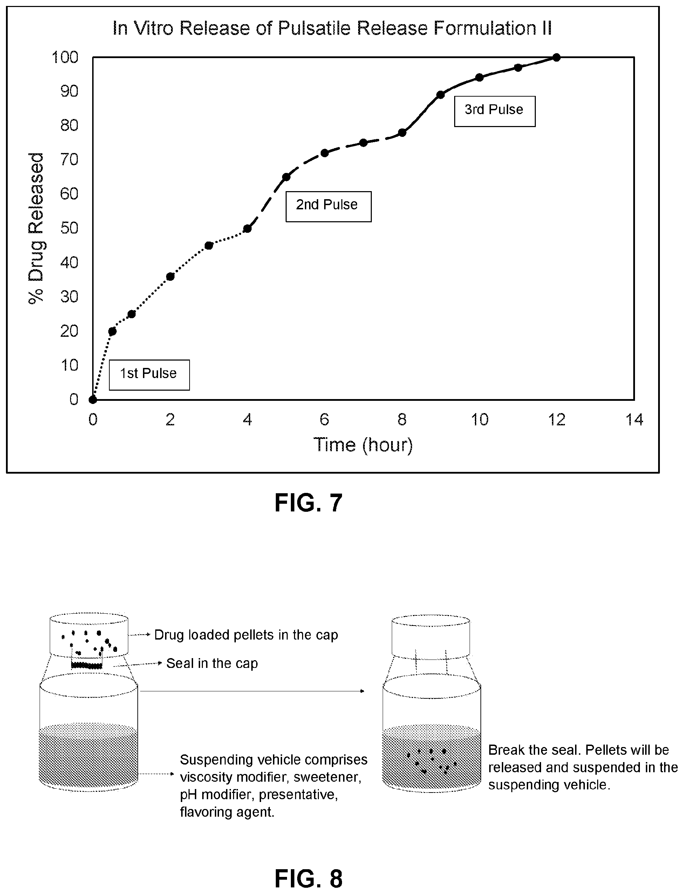

Pulsatile drug release can release equal amount of drug during each pulse (see **Table 31 &** **FIG. 6**), or can release different amount of drug during each pulse (see **Table 32 &** **FIG. 7**), or can release for different extended periods during each pulse.

**Table 31. Pulsatile Release Formulation I**

| Pulsatile Release | | |
|---|---|---|
| # | Ingredients | Solids Quantity (mg) |
| Granules I (1^{st} pulse) | Droxidopa | 300 |
| | Hydroxylpropyl cellulose | 20 |
| | Microcrystalline cellulose | 100 |
| | Monosodium citrate | 35 |
| | Purified water | q.s. |
| Granules II (2^{nd} pulse) | Droxidopa | 300 |
| | Hypromellose | 50 |
| | Microcrystalline cellulose | 100 |
| | Hydrogenated castor oil | 300 |
| | Purified water | q.s. |
| | Eudragit L100 | 150 |
| | Isopropyl Alcohol | q.s. |
| Granules III (3^{rd} pulse) | Droxidopa | 300 |
| | Hypromellose | 50 |
| | Microcrystalline cellulose | 100 |
| | Hydrogenated castor oil | 300 |
| | Purified water | q.s. |
| | Eudragit S100 | 150 |
| | Isopropyl Alcohol | q.s. |
| TOTAL | | 2255 |

**Table 32. Pulsatile Release Formulation II**

| Pulsatile Release | | |
|---|---|---|
| # | Ingredients | Solids Quantity (mg) |
| Granules I (1st pulse) | Droxidopa | 450 |
| | Hydroxylpropyl cellulose | 30 |
| | Microcrystalline cellulose | 150 |
| | Monosodium citrate | 50 |
| | Purified water | q.s. |
| Granules II (2nd pulse) | Droxidopa | 250 |
| | Hypromellose | 50 |
| | Microcrystalline cellulose | 100 |
| | Hydrogenated castor oil | 250 |
| | Purified water | q.s. |
| | Eudragit L100 | 150 |
| | Isopropyl Alcohol | q.s. |
| Granules III (3rd pulse) | Droxidopa | 200 |
| | Hypromellose | 35 |
| | Microcrystalline cellulose | 70 |
| | Hydrogenated castor oil | 200 |
| | Purified water | q.s. |
| | Eudragit S100 | 150 |
| | Isopropyl Alcohol | q.s. |
| TOTAL | | 2135 |

### Example 18: The chemical stability of droxidopa suspensions

Droxidopa pellet suspensions were prepared and stored at room temperature and at refrigerated temperature for up to 21 days in sealed containers. Suspensions included pellets containing 900 mg droxidopa (prepared similarly to pellets I of Formulation II in Example 16: 900 mg droxidopa; 900 mg microcrystalline cellulose; water, q.s.) in 30 ml of a suspending vehicle as shown below. A description of the study is shown in **Table 33.**

**Table 33**

| Study number | Sample Description | Pellets with 50% drug load at 900mg dose (mg) | Monosodium citrate (mg) | Xanthan gum (mg) | Sodium benzoate (mg) |
|---|---|---|---|---|---|
| 1 | Room temperature | 1800 | 85 | 90 | 100 |
| 2 | Room temperature | 1800 | 0 | 90 | 100 |
| 3 | Refrigerated at 4C | 1800 | 85 | 90 | 100 |
| 4 | Refrigerated at 4C | 1800 | 0 | 90 | 100 |

For tested samples, the suspension was centrifuged and the supernatant was diluted 1 to 5 with water. Supernatant of the suspension was analyzed. Degradation as a percentage of droxidopa amount was determined by ultra-performance liquid chromatography (UPLC) on a Waters ACQUITY UPLC with a photodiode array (PDA) detector utilizing 2 methods that covered the range of separation characteristics for potential degradation products.

Method 1: Analysis performed using a Waters Acquity HSS PFP 1.8µm 2.1x100 mm column. Run conditions for the analyses were as follows:
Mobile phase A: 10mM ammonium formate adjusted to pH 3.0 with formic acid
Mobile phase B: acetonitrile
Detection wavelength: 279 nm
Flow: 0.5 ml/min
Column temperature: 30 °C
Injection volume: 1 µl
Gradient (linear transitions) are shown in **Table 34:**

**Table 34**

| Time, minutes | % Mobile Phase B |
|---|---|
| Initial | 0 |
| 1.2 | 0 |
| 5.0 | 20 |
| 6.0 | 20 |
| 6.1 | 0 |
| 8.5 | 0 |

Method 2: Analysis performed using a Waters Acquity BEH Amide 1.7µm 2.1x50 mm column. Run conditions for the analyses were as follows:
Mobile phase: 23:77 v/v 80mM ammonium formate adjusted to pH 3.0 with formic acid: acetonitrile
Detection wavelength: 281 nm
Flow: 0.6 ml/min isocratic
Run time: 5 minutes
Column temperature: 30 °C
Injection volume: 0.5 µl

Identification of a known degradation product dihydroxybenzaldehyde was confirmed through retention time and spectral match and quantified against pure material of known identity. Degradation results for unidentified peaks were quantified against a droxidopa standard of known concentration assuming a relative response of 1.

**Table 35** below summarizes stability data for droxidopa after exposure in suspended solution.

**Table 35**

| Study Number | 21 Day Exposure | |
|---|---|---|
| | Major Degradant¹, % | Total Degradation, % |
| 1 | 0.01 | 0.01 |
| 2 | 0.20 | 0.4 |
| 3 | 0.00 | 0.0 |
| 4 | 0.02 | 0.1 |

| | | |
|---|---|---|
| ¹Dihydroxybenzaldehyde | | |

These results showed that the presence of a buffering agent (monosodium citrate) stabilized the droxidopa pellet suspension stored at room temperature for at least 21 days.

## Claims

1. An extended-release liquid composition for oral administration comprising:
(a)
(i) a first multi-particulate comprising droxidopa, or a pharmaceutically acceptable salt thereof, and optionally a first release controlling agent; and
(ii) a second multi-particulate comprising droxidopa, or a pharmaceutically acceptable salt thereof, a second release controlling agent, and an enteric layer; and
(b) a liquid vehicle,
wherein the first multi-particulate is capable of releasing droxidopa, or a pharmaceutically acceptable salt thereof, at a pH of less than 1.5, and the second multi-particulate is capable of releasing droxidopa, or a pharmaceutically acceptable salt thereof, at a pH of 5.5

2. The liquid compostition of claim 1, wherein the liquid vehicle has a pH less than 7.0.

3. The liquid compostition of claim 1 or claim 2, wherein the first multi-particulate comprises 20% to 40% of the total amount of droxidopa, or a pharmaceutically acceptable salt thereof, in the composition; and/or, wherein the second multi-particulate comprises 60% to 80% of the total amount of droxidopa, or a pharmaceutically acceptable salt thereof, in the composition.

4. The liquid compostition of any of claims 1 to 3, wherein the liquid vehicle comprises a pH modifier; and wherein the pH modifier is present in an amount sufficient to increase stability of droxidopa in the composition.

5. The liquid composition of any of the preceding claims, wherein the composition is a suspension.

6. The liquid composition or the suspension of any of the preceding claims, wherein the total amount of droxidopa, or a pharmaceutically-acceptable salt thereof, in a single 5 mL to 100 mL dose of the liquid composition or the suspension is 100 mg to 3000 mg, optionally 100 mg to 1800 mg.

7. A solid pharmaceutical composition for oral administration comprising
(i) a first multi-particulate comprising droxidopa, or a pharmaceutically acceptable salt thereof, and optionally a first release controlling agent; and
(ii) a second multi-particulate comprising droxidopa, or a pharmaceutically acceptable salt thereof, a second release controlling agent, and an enteric layer,
wherein the first multi-particulate is capable of releasing droxidopa, or a pharmaceutically acceptable salt thereof, at a pH of less than 1.5, and the second multi-particulate is capable of releasing droxidopa, or a pharmaceutically acceptable salt thereof, at a pH of 5.5

8. The solid composition of claim 7, wherein the solid composition is packaged in a bottle or a sachet for suspension in a liquid vehicle.

9. The solid composition of claim 7, wherein the first and/or second multi-particulate further comprises a pH-controlling agent.

10. The compostition of any preceding claim, wherein the first release controlling agent is a non-polymeric material, and/or, wherein the second release controlling agent is a non-polymeric material.

11. The composition of any preceding claim, wherein the first multi-particulate is coated with a moisture protection layer.

12. The composition of any preceding claim, further comprising ≤ 14% or ≥56% of the total amount of droxidopa, or a pharmaceutically acceptable salt thereof, in the composition in an immediate release form.

13. The composition of any one of the preceding claims, wherein the composition is packaged to provide per package a daily dosage amount of droxidopa, or the pharmaceutically acceptable salt thereof, of 100 mg to 3000 mg, optionally 100 mg to 1800 mg.

14. A pharmaceutical kit comprising the solid composition of claim 7, and a liquid vehicle,
optionally in the form of a capped bottle wherein the composition is stored in a compartment within the cap of the bottle; and the liquid vehicle is stored in the bottle.

15. The composition of any one of claims 1 to 13, or the pharmaceutical kit of claim 14, for use in treatment of hypotension, neurogenic orthostatic hypotension (nOH), intradialytic hypotension, a symptom of Parkinson's disease, orthostatic hypotension associated with Parkinson's disease, postural instability associated with Parkinson's disease, postural orthostatic tachycardia syndrome (POTS), Down's syndrome, a demyelinating disease, Alzheimer's disease, an attention deficit disorder, hypersomnia, pain associated with fibromyalgia, motor paralysis, motor aphasia, urinary incontinence, dementia, antidiuresis, postural tachycardia syndrome, tauopathy, fatigue, headaches, neurological deficits or neuronal death induced by brain ischemia, intracranial hypertension or cerebral edema, cancer, a bacterial infection, to induce or facilitate micturition, nasal congestion, acute pain, chronic pain, or any combination thereof.

## Patentansprüche

1. Flüssige Zusammensetzung mit verlängerter Wirkstofffreisetzung zur oralen Verabreichung, die Folgendes umfasst:
(a)
(i) ein erstes Multipartikulat, das Droxidopa oder ein pharmazeutisch verträgliches Salz davon und optional ein erstes Freisetzungskontrollmittel umfasst; und
(ii) ein zweites Multipartikulat, das Droxidopa oder ein pharmazeutisch verträgliches Salz davon, ein zweites Freisetzungskontrollmittel und eine magensaftresistente Schicht umfasst; und
(b) ein flüssiges Vehikel,
wobei das erste Multipartikulat in der Lage ist, Droxidopa oder ein pharmazeutisches verträgliches Salz davon bei einem pH-Wert von weniger als 1,5 freizusetzen und das zweite Multipartikulat in der Lage ist, Droxidopa oder ein pharmazeutisch verträgliches Salz davon bei einem pH-Wert von 5,5 freizusetzen.

2. Flüssige Zusammensetzung nach Anspruch 1, wobei das flüssige Vehikel einen pH-Wert von weniger als 7,0 aufweist.

3. Flüssige Zusammensetzung nach Anspruch 1 oder Anspruch 2, wobei das erste Multipartikulat 20 % bis 40 % der Gesamtmenge an Droxidopa oder einem pharmazeutisch verträglichen Salz davon in der Zusammensetzung umfasst; und/oder wobei das zweite Multipartikulat 60 % bis 80 % der Gesamtmenge an Droxidopa oder einem pharmazeutisch verträglichen Salz davon in der Zusammensetzung umfasst.

4. Flüssige Zusammensetzung nach einem der Ansprüche 1 bis 3, wobei das flüssige Vehikel einen pH-Modifikator umfasst; und wobei der pH-Modifikator in einer Menge vorhanden ist, die ausreicht, um die Stabilität von Droxidopa in der Zusammensetzung zu erhöhen.

5. Flüssige Zusammensetzung nach einem der vorhergehenden Ansprüche, wobei die Zusammensetzung eine Suspension ist.

6. Flüssige Zusammensetzung oder Suspension nach einem der vorhergehenden Ansprüche, wobei die Gesamtmenge an Droxidopa oder einem pharmazeutisch verträglichen Salz davon in einer Einzeldosis von 5 mL bis 100 mL der flüssigen Zusammensetzung oder der Suspension 100 mg bis 3000 mg, optional 100 mg bis 1800 mg beträgt.

7. Feste pharmazeutische Zusammensetzung zur oralen Verabreichung, die Folgendes umfasst:
(i) ein erstes Multipartikulat, das Droxidopa oder ein pharmazeutisch verträgliches Salz davon und optional ein erstes Freisetzungskontrollmittel umfasst; und
(ii) ein zweites Multipartikulat, das Droxidopa oder ein pharmazeutisch verträgliches Salz davon, ein zweites Freisetzungskontrollmittel und eine magensaftresistente Schicht umfasst,
wobei das erste Multipartikulat in der Lage ist, Droxidopa oder ein pharmazeutisch verträgliches Salz davon bei einem pH-Wert von weniger als 1,5 freizusetzen und das zweite Multipartikulat in der Lage ist, Droxidopa oder ein pharmazeutisch verträgliches Salz davon bei einem pH-Wert von 5,5 freizusetzen.

8. Feste Zusammensetzung nach Anspruch 7, wobei die feste Zusammensetzung in einer Flasche oder einem Beutel zur Suspension in einem flüssigen Vehikel verpackt ist.

9. Feste Zusammensetzung nach Anspruch 7, wobei das erste und/oder zweite Multipartikulat ferner ein pH-Kontrollmittel umfassen.

10. Zusammensetzung nach einem der vorhergehenden Ansprüche, wobei das erste Freisetzungskontrollmittel ein nichtpolymeres Material ist und/oder wobei das zweite Freisetzungskontrollmittel ein nichtpolymeres Material ist.

11. Zusammensetzung nach einem der vorhergehenden Ansprüche, wobei das erste Multipartikulat mit einer Feuchtigkeitsschutzschicht überzogen ist.

12. Zusammensetzung nach einem der vorhergehenden Ansprüche, die ferner ≤ 14 % oder ≥56 % der Gesamtmenge an Droxidopa oder einem pharmazeutisch verträglichen Salz davon in der Zusammensetzung in einer Form mit sofortiger Freisetzung umfasst.

13. Zusammensetzung nach einem der vorhergehenden Ansprüche, wobei die Zusammensetzung so verpackt ist, dass sie pro Packung eine tägliche Dosierungsmenge an Droxidopa oder dem pharmazeutisch verträglichen Salz davon von 100 mg bis 3000 mg, optional 100 mg bis 1800 mg bereitstellt.

14. Pharmazeutisches Kit, das die feste Zusammensetzung nach Anspruch 7 und ein flüssiges Vehikel umfasst,
optional in Form einer verschlossenen Flasche, wobei die Zusammensetzung in einem Fach im Verschluss der Flasche aufbewahrt wird; und das flüssige Vehikel in der Flasche aufbewahrt wird.

15. Zusammensetzung nach einem der Ansprüche 1 bis 13 oder pharmazeutisches Kit nach Anspruch 14 zur Verwendung bei der Behandlung von Hypotonie, neurogener orthostatischer Hypotonie (nOH), intradialytischer Hypotonie, einem Symptom der Parkinson-Krankheit, orthostatischer Hypotonie im Zusammenhang mit der Parkinson-Krankheit, posturaler Instabilität im Zusammenhang mit der Parkinson-Krankheit, posturalem orthostatischem Tachykardie-Syndrom (POTS), Down-Syndrom, einer demyelinisierenden Krankheit, Alzheimer-Krankheit, Aufmerksamkeitsdefizitstörung, Hypersomnie, Schmerzen im Zusammenhang mit Fibromyalgie, motorischer Lähmung, motorischer Aphasie, Harninkontinenz, Demenz, Antidiurese, posturalem Tachykardie-Syndrom, Tauopathie, Müdigkeit, Kopfschmerzen, neurologischen Defiziten oder neuronalem Tod durch Hirnischämie, intrakranialer Hypertonie oder Hirnödem, Krebs, einer bakteriellen Infektion, zur Einleitung oder Erleichterung der Miktion, verstopfter Nase, akuten Schmerzen, chronischen Schmerzen oder einer Kombination davon.

## Revendications

1. Composition liquide à libération prolongée pour administration orale comprenant :
(a)
(i) une première composition multiparticulaire comprenant de la droxidopa, ou un sel pharmaceutiquement acceptable de celle-ci, et éventuellement un premier agent de contrôle de libération ; et
(ii) une deuxième composition multiparticulaire comprenant de la droxidopa, ou un sel pharmaceutiquement acceptable de celle-ci, un deuxième agent de contrôle de libération, et une couche entérique ; et
(b) un véhicule liquide,
où la première composition multiparticulaire est capable de libérer de la droxidopa, ou un sel pharmaceutiquement acceptable de celle-ci, à un pH inférieur à 1,5, et la deuxième composition multiparticulaire est capable de libérer de la droxidopa, ou un sel pharmaceutiquement acceptable de celle-ci, à un pH de 5,5.

2. Composition liquide selon la revendication 1, où le véhicule liquide a un pH inférieur à 7,0.

3. Composition liquide selon la revendication 1 ou la revendication 2, où la première composition multiparticulaire comprend 20 % à 40 % de la quantité totale de droxidopa, ou d'un sel pharmaceutiquement acceptable de celle-ci, dans la composition ; et/ou, où la deuxième composition multiparticulaire comprend 60 % à 80 % de la quantité totale de droxidopa, ou d'un sel pharmaceutiquement acceptable de celle-ci, dans la composition.

4. Composition liquide selon l'une quelconque des revendications 1 à 3, où le véhicule liquide comprend un modificateur de pH ; et où le modificateur de pH est présent en une quantité suffisante pour augmenter la stabilité de la droxidopa dans la composition.

5. Composition liquide selon l'une quelconque des revendications précédentes, où la composition est une suspension.

6. Composition liquide ou suspension selon l'une quelconque des revendications précédentes, où la quantité totale de droxidopa, ou d'un sel pharmaceutiquement acceptable de celle-ci, dans une dose unique de 5 ml à 100 ml de la composition liquide ou de la suspension est de 100 mg à 3000 mg, éventuellement de 100 mg à 1800 mg.

7. Composition pharmaceutique solide pour administration orale comprenant
(i) une première composition multiparticulaire comprenant de la droxidopa, ou un sel pharmaceutiquement acceptable de celle-ci, et éventuellement un premier agent de contrôle de libération ; et
(ii) une deuxième composition multiparticulaire comprenant de la droxidopa, ou un sel pharmaceutiquement acceptable de celle-ci, un deuxième agent de contrôle de libération et une couche entérique,
où la première composition multiparticulaire est capable de libérer de la droxidopa, ou un sel pharmaceutiquement acceptable de celle-ci, à un pH inférieur à 1,5, et la deuxième composition multiparticulaire est capable de libérer de la droxidopa, ou un sel pharmaceutiquement acceptable de celle-ci, à un pH de 5,5.

8. Composition solide selon la revendication 7, où la composition solide est conditionnée dans un flacon ou un sachet pour suspension dans un véhicule liquide.

9. Composition solide selon la revendication 7, où la première et/ou la deuxième composition multiparticulaire comprend en outre un agent de contrôle de pH.

10. Composition selon l'une quelconque des revendications précédentes, où le premier agent de contrôle de libération est un matériau non polymère, et/ou où le deuxième agent de contrôle de libération est un matériau non polymère.

11. Composition selon l'une quelconque des revendications précédentes, où la première composition multiparticulaire est recouverte d'une couche de protection contre l'humidité.

12. Composition selon l'une quelconque des revendications précédentes, comprenant en outre ≤ 14 % ou ≥ 56 % de la quantité totale de droxidopa, ou d'un sel pharmaceutiquement acceptable de celle-ci, dans la composition sous une forme à libération immédiate.

13. Composition selon l'une quelconque des revendications précédentes, où la composition est conditionnée pour fournir par emballage une quantité quotidienne de dosage de droxidopa, ou d'un sel pharmaceutiquement acceptable de celle-ci, de 100 mg à 3000 mg, éventuellement de 100 mg à 1800 mg.

14. Kit pharmaceutique comprenant la composition solide selon la revendication 7 et un véhicule liquide,
éventuellement sous la forme d'un flacon muni d'un bouchon, où la composition est stockée dans un compartiment à l'intérieur du bouchon du flacon ; et le véhicule liquide est stocké dans le flacon.

15. Composition selon l'une quelconque des revendications 1 à 13, ou le kit pharmaceutique selon la revendication 14, pour utilisation dans le traitement de l'hypotension, de l'hypotension orthostatique neurogène (nOH), de l'hypotension intradialytique, d'un symptôme de la maladie de Parkinson, de l'hypotension orthostatique associée à la maladie de Parkinson, de l'instabilité posturale associée à la maladie de Parkinson, du syndrome de tachycardie orthostatique posturale (POTS), du syndrome de Down, d'une maladie démyélinisante, de la maladie d'Alzheimer, d'un trouble déficitaire de l'attention, de l'hypersomnie, de la douleur associée à la fibromyalgie, de la paralysie motrice, de l'aphasie motrice, de l'incontinence urinaire, de la démence, de l'antidiurèse, du syndrome de tachycardie posturale, de la tauopathie, de la fatigue, des maux de tête, des déficits neurologiques ou de la mort neuronale induits par une ischémie cérébrale, de l'hypertension intracrânienne ou de l'œdème cérébral, du cancer, d'une infection bactérienne, pour induire ou faciliter la miction, de la congestion nasale, de la douleur aiguë, de la douleur chronique, ou toute combinaison de ceux-ci.
